# EUROPEAN PATENT SPECIFICATION

(11) **EP 4 076 665 B1**
(45) Date of publication and mention of the grant of the patent: **30.08.2023**
(21) Application number: 20835790.5
(22) Date of filing: 18.12.2020
(51) Int. Cl.: A61P 35/00, C07D 487/04, A61K 31/454, A61K 31/427, A61K 31/55

(54) **EGFR INHIBITORS**
EGFR-INHIBITOREN
INHIBITEURS D' EGFR

(30) Priority: 20.12.2019 EP 19218390
(43) Date of publication of application: 26.10.2022
(73) Proprietor: F. Hoffmann-La Roche AG, 4070 Basel (CH)
(72) Inventor: HEWINGS, David Stephen, 4070 Basel (CH); JAESCHKE, Georg, 4070 Basel (CH); KUHN, Bernd, 4070 Basel (CH); NAGEL, Yvonne Alice, 4070 Basel (CH); RICCI, Antonio, 4070 Basel (CH)
(74) Representative: Salud, Carlos E.
(86) International application number: PCT/EP2020/086917
(87) International publication number: WO 2021/123087

(56) References cited:
- WO-A1-2017/004383
- WO-A1-2018/115218
- WO-A1-2018/220149
- WO-A1-2019/149922
- YONG JIA ET AL: "Overcoming EGFR(T790M) and EGFR(C797S) resistance with mutant-selective allosteric inhibitors", NATURE, vol. 534, no. 7605, 25 May 2016 (2016-05-25), pages 129-132, XP055342543, London ISSN: 0028-0836, DOI: 10.1038/nature17960

## Description

The present invention provides a compound that is selective allosteric inhibitors of T790M/L858R, T790M/L858R/C797S, L858R, L858R/C797S containing EGFR mutants, its manufacture and pharmaceutical compositions containing it. Disclosed but not claimed is its use as a therapeutically active substance.

The present invention provides a novel compound of formula (I) 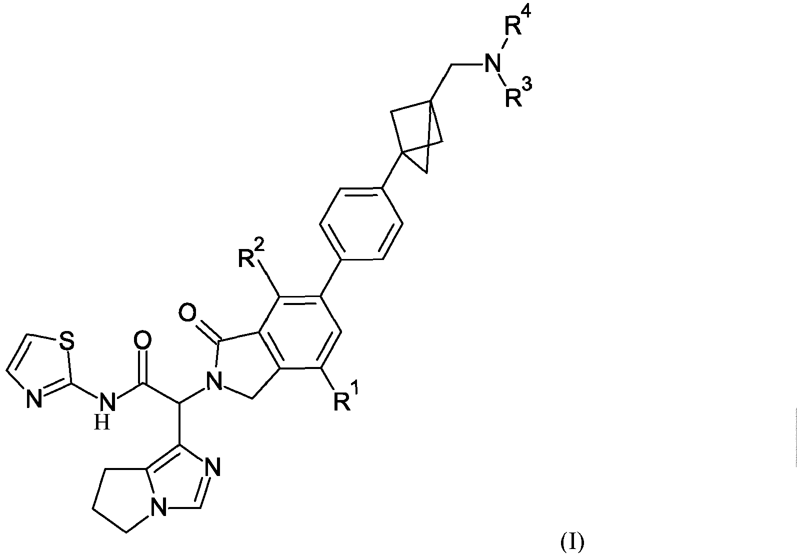 wherein
R¹ is selected from
   i) fluoro, and
   ii) chloro;
R² is selected from
   i) H, and
   ii) fluoro;
R³ is C₁₋₆-alkyl;
R⁴ is C₁₋₆-alkyl;
or R³ and R⁴ together with the nitrogen atom to which they are attached form an heterocycloalkyl optionally substituted with R⁵; and
R⁵ is selected from
   i) hydroxy, and
   ii) hydrox-C₁₋₆-alkyl;
or a pharmaceutically acceptable salt thereof.

The HER family receptor tyrosine kinases are mediators of cell growth, differentiation and survival. The receptor family includes four distinct members, i.e. epidermal growth factor receptor (EGFR, ErbBl, or HER1) HER2 (ErbB2), HER3 (ErbB3) and HER4 (ErbB4). Upon ligand binding the receptors form homo and heterodimers and subsequent activation of the intrinsic tyrosine kinase activity leads to receptor auto-phosphorylation and the activation of downstream signaling molecules (Yarden, Y., Sliwkowski, MX. Untangling the ErbB signalling network. Nature Review Mol Cell Biol. 2001 Feb;2(2): 127-37). De-regulation of EGFR by overexpression or mutation has been implicated in many types of human cancer including colorectal, pancreatic, gliomas, head and neck and lung cancer, in particular non-small cell lung cancer (NSCLC) and several EGFR targeting agents have been developed over the years (Ciardiello, F., and Tortora, G. (2008). EGFR antagonists in cancer treatment. The New England journal of medicine 358, 1160-1174). Erlotinib (Tarceva^{®}), a reversible inhibitor of the EGFR tyrosine kinase was approved in numerous countries for the treatment of recurrent NSCLC.

An impressive single agent activity of EGFR tyrosine kinase inhibitors is observed in a subset of NSCLC patients whose tumors harbor somatic kinase domain mutations, whereas clinical benefit in wild-type EGFR patients is greatly diminished (Paez, J. et al. (2004). EGFR mutations in lung cancer: correlation with clinical response to gefitinib therapy. Science (New York, NY 304, 1497-1500). The most common somatic mutations of EGFR are exon 19 deletions with delta 746-750 the most prevalent mutation and the exon 21 amino acid substitutions with L858R the most frequent mutation (Sharma SV, Bell DW, Settleman J, Haber DA. Epidermal growth factor receptor mutations in lung cancer. Nat Rev Cancer. 2007 Mar;7(3): 169-81).

Treatment resistance arises frequently, often due to the secondary T790M mutation within the ATP site of the receptor. Some developed mutant-selective irreversible inhibitors are highly active against the T790M mutant, but their efficacy can be compromised by acquired mutation of C797S, that is the cysteine residue with which they form a key covalent bond (Thress, K. S. et al. Acquired EGFR C797S mutation mediates resistance to AZD9291 in non-small cell lung cancer harboring EGFR T790M. Nat. Med. 21, 560-562 (2015)). C797S mutation was further reported by Wang to be a major mechanism for resistance to T790M-targeting EGFR inhibitors (Wang et al. EGFR C797S mutation mediates resistance to third-generation inhibitors in T790M-positive non-small cell lung cancer, J Hematol Oncol. 2016; 9: 59). Additional mutations that cause resistance to Osimertinib are described by Yang, for example L718Q ( Yang et al, Investigating Novel Resistance Mechanisms to Third-Generation EGFR Tyrosine Kinase Inhibitor Osimertinib in Non-Small Cell Lung Cancer Patients, Clinical Cancer Research, DOI: 10.1158/1078-0432.CCR-17-2310). Lu et al.( Targeting EGFRL858R/T790M and EGFRL858R/T790M/C797S resistance mutations in NSCLC: Current developments in medicinal chemistry, Med Res Rev 2018; 1-32) report in a review article on Targeting EGFR^{L858R/T790M} and EGFR^{L858R/T790M/C797S} resistance mutations in NSCLC treatment.

As most available EGFR tyrosine kinase inhibitors target the ATP-site of the kinase, there is a need for new therapeutic agents that work differently, for example through targeting drug-resistant EGFR mutants.

Recent studies suggest that purposefully targeting allosteric sites might lead to mutant-selective inhibitors (Jia et al. Overcoming EGFR(T790M) and EGFR(C797S) resistance with mutant-selective allosteric inhibitors, June 2016, Nature 534, 129-132).

There is just a need in the generation of selective molecules that specifically inhibit T790M/L858R, T790M/L858R/C797S, L858R, L858R/C797S containing EGFR mutants useful for the therapeutic and/or prophylactic treatment of cancer, in particular T790M and C797S containing EGFR mutants. Such inhibitors have been disclosed, for example, in WO 2018/220149.

The compound of formula (I) as described herein does have improved EGFR potency and selectivity for T790M/L858R, T790M/L858R/C797S, L858R, L858R/C797S containing EGFR mutants, in particular T790M and C797S containing EGFR mutants as well as improved physicochemical properties.

The term "C₁₋₆-alkyl" denotes a monovalent linear or branched saturated hydrocarbon group of 1 to 6 carbon atoms, particularly 1 to 3 carbon atoms. Examples of C₁₋₆-alkyl include methyl, ethyl, n-propyl, isopropyl, n-butyl, iso-butyl, sec-butyl, tert-butyl and pentyl. Particular C₁₋₆-alkyl groups are methyl, ethyl and isopropyl. More particular example is methyl.

The term "heterocycloalkyl" denotes a monovalent saturated or partly unsaturated mono- or bicyclic ring system of 4 to 9 ring atoms, comprising 1, 2, or 3 ring heteroatoms selected from N, O and S, the remaining ring atoms being carbon. Bicyclic means consisting of two cycles having one or two ring atoms in common. Examples for monocyclic saturated heterocycloalkyl are 4,5-dihydro-oxazolyl, oxetanyl, azetidinyl, pyrrolidinyl, 2-oxo-pyrrolidin-3-yl, tetrahydrofuranyl, tetrahydrothiophenyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Examples for bicyclic saturated heterocycloalkyl are oxabicyclo[2.2.1]heptanyl, oxaspiro[3.3]heptanyl, 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, or 3-thia-9-aza-bicyclo[3.3.1]nonyl. Examples for partly unsaturated heterocycloalkyl are dihydrofuryl, imidazolinyl, dihydro-oxazolyl, tetrahydro-pyridinyl, or dihydropyranyl. Particular particular heterocycloalkyl is piperidinyl, pyrrolidinyl, and azepanyl.

The term "hydroxy" denotes a -OH group.

The term "hydroxy-C₁₋₆-alkyl alkyl" denotes an C₁₋₆-alkyl alkyl group wherein at least one of the hydrogen atoms of the C₁₋₆-alkyl alkyl group has been replaced by a hydroxy group. Examples of hydroxy-C₁₋₆-alkyl include hydroxymethyl, hydroxyethyl and hydroxypropyl. Particular example is hydroxymentyl.

The term "pharmaceutically acceptable salt" refers to those salts of the compound of formula (I) which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein and the like. In addition, these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compound of formula (I) are the hydrochloride salts, methanesulfonic acid salts and citric acid salts.

The abbreviation uM means microMolar and is equivalent to the symbol µM.

The abbreviation uL means microliter and is equivalent to the symbol µL.

The abbreviation ug means microgram and is equivalent to the symbol µg.

The compound of formula (I) can contain several asymmetric centers and can be present in the form of optically pure enantiomers, mixtures of enantiomers such as, for example, racemates, optically pure diastereoisomers, mixtures of diastereoisomers, diastereoisomeric racemates or mixtures of diastereoisomeric racemates.

According to the Cahn-Ingold-Prelog Convention the asymmetric carbon atom can be of the "R" or "S" configuration.

Also an embodiment of the present invention is a compound according to formula (I) as described herein and pharmaceutically acceptable salts thereof, in particular a compound according to formula (I) as described herein.

Also an embodiment of the present invention is a compound according to formula (I) as described herein.

A particular embodiment of the present invention provides a compound according to formula (I) as described herein, wherein
R¹ is selected from
   i) fluoro, and
   ii) chloro;
R² is selected from
   i) H, and
   ii) fluoro;
R³ and R⁴ are methyl;
or R³ and R⁴ together with the nitrogen atom to which they are attached form an heterocycloalkyl optionally substituted with R⁵, wherein the heterocycloalkyl is selected from
   i) piperidinyl,
   ii) pyrrolidinyl, and
   iii) azepanyl;
R⁵ is selected from
   i) hydroxy, and
   ii) hydroxymethyl;
or a pharmaceutically acceptable salt thereof.

A further particular embodiment of the present invention provides a compound according to formula (I) as described herein, wherein
R¹ is fluoro;
   R² is selected from
   i) H, and
   ii) fluoro;
R³ and R⁴ together with the nitrogen atom to which they are attached form an heterocycloalkyl optionally substituted with R⁵, wherein the heterocycloalkyl is selected from
   i) piperidinyl,
   ii) pyrrolidinyl, and
   iii) azepanyl;
R⁵ is selected from
   i) hydroxy, and
   ii) hydroxymethyl;
or a pharmaceutically acceptable salt thereof.

A particular embodiment of the present invention provides a compound according to formula (I) as described herein, wherein R¹ is fluoro.

A particular embodiment of the present invention provides a compound according to formula (I) as described herein, wherein R¹ is fluoro.

A particular embodiment of the present invention provides a compound according to formula (I) as described herein, wherein R² is H.

A particular embodiment of the present invention provides a compound according to formula (I) as described herein, wherein
R³ and R⁴ together with the nitrogen atom to which they are attached form an heterocycloalkyl optionally substituted with R⁵, wherein the heterocycloalkyl is selected from
i) piperidinyl,
ii) pyrrolidinyl, and
iii) azepanyl.

A particular embodiment of the present invention provides a compound according to formula (I) as described herein, wherein the compound is selected from
(2RS)-2-(6,7-dihydro-5H-pyrrolo[1,2-c]imidazol-1-yl)-2-[4-chloro-6-[4-[3-[[4-(hydroxymethyl)-1-piperidyl]methyl]-1-bicyclo[1.1.1]pentanyl]phenyl]-1-oxo-isoindolin-2-yl]-N-thiazol-2-yl-acetamide;
(2RS)-2-(6,7-dihydro-5H-pyrrolo[1,2-c]imidazol-1-yl)-2-[4-fluoro-6-[4-[3-[[4-(hydroxymethyl)-1-piperidyl]methyl]-1-bicyclo[1.1.1]pentanyl]phenyl]-1-oxo-isoindolin-2-yl]-N-thiazol-2-yl-acetamide;
(2RS)-2-(6,7-dihydro-5H-pyrrolo[1,2-c]imidazol-1-yl)-2-[6-[4-[3-[(dimethylamino)methyl]-1-bicyclo[1.1.1]pentanyl]phenyl]-4-fluoro-1-oxo-isoindolin-2-yl]-N-thiazol-2-yl-acetamide;
(2RS)-2-(6,7-dihydro-5H-pyrrolo[1,2-c]imidazol-1-yl)-2-[4-fluoro-6-[4-[3-[(4-hydroxy-1-piperidyl)methyl]-1-bicyclo[1.1.1]pentanyl]phenyl]-1-oxo-isoindolin-2-yl]-N-thiazol-2-yl-acetamide;
(2RS)-2-(6,7-dihydro-5H-pyrrolo[1,2-c]imidazol-1-yl)-2-[4-fluoro-1-oxo-6-[4-[3-(pyrrolidin-1-ylmethyl)-1-bicyclo[1.1.1]pentanyl]phenyl]isoindolin-2-yl]-N-thiazol-2-yl-acetamide;
(2RS)-2-(6,7-dihydro-5H-pyrrolo[1,2-c]imidazol-1-yl)-2-[4-fluoro-6-[4-[3-[[(4RS)-4-hydroxyazepan-1-yl]methyl]-1-bicyclo[1.1.1]pentanyl]phenyl]-1-oxo-isoindolin-2-yl]-N-thiazol-2-yl-acetamide; and
(2RS)-2-[4,7-difluoro-6-[4-[3-[[4-(hydroxymethyl)-1-piperidyl]methyl]-1-bicyclo[1.1.1]pentanyl]phenyl]-1-oxo-isoindolin-2-yl]-2-(6,7-dihydro-5H-pyrrolo[1,2-c]imidazol-1-yl)-N-thiazol-2-yl-acetamide;
or a pharmaceutically acceptable salt thereof.

The invention thus also relates to a process for the preparation of a compound according to the invention, comprising the coupling of a compound of formula (B1)

The invention thus also relates to a process for the preparation of a compound according to the invention, comprising the reaction of a compound of formula (B1) or a pharmaceutically acceptable salt thereof 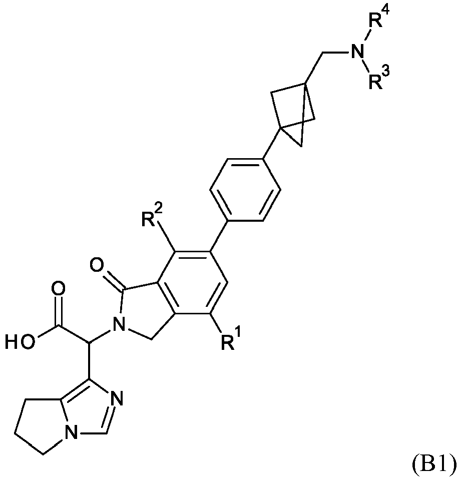 with a compound of formula (B2) 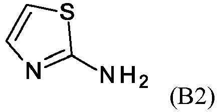 in the presence of a base and a coupling agent.

The reaction can conveniently be carried out in a solvent. The solvent can be for example DMF.

In the reaction the base can be for example Hunig's base, trimethylamine, triethylamine, dimethylamine or diethylamine. Conveniently the base is Hunig's base.

In the reaction the coupling agent can be for example HATU.

Convenient conditions for the reaction can be between around 5°C to around 60°C, particularly between around 10°C to around 50°C, more particularly between around 15°C to around 40°C.

Preferred conditions for the reaction are the use of Hunig's base and HATU in DMF at around 25°C for between around 1h to around 48 hrs, in particular between around 1h to around 16 hrs.

Processes for the manufacture of a compound of formula (I) as described herein are also an object of the invention.

The preparation of compound of formula (I) of the present invention may be carried out in sequential or convergent synthetic routes. Syntheses of the invention are shown in the following general scheme. The skills required for carrying out the reactions and purifications of the resulting products are known to those skilled in the art. The substituents and indices used in the following description of the processes have the significance given herein before unless indicated to the contrary.

In more detail, the compound of formula (I) can be manufactured by the methods given below, by the methods given in the examples or by analogous methods. Appropriate reaction conditions for the individual reaction steps are known to a person skilled in the art. The reaction sequence is not limited to the one displayed in scheme 1, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

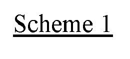

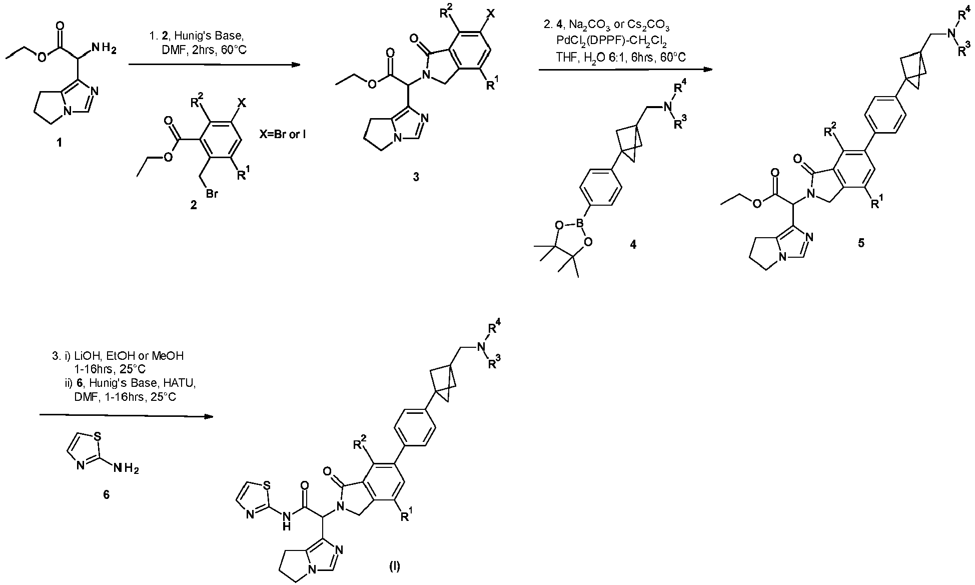

A compound of general formula (I) can be obtained for example by ring cyclization of a previously prepared aminoester **1** with an appropiately substituted bromomethylbenzoate of formula **2** to yield the desired isoindoline ester **3**. Suzuki coupling of **3** with an appropiate substituted bornonic ester of formula **4** yields the desired compound of formula **5**. Saponification of the ester **5** and amide coupling with 2-aminothiazole **6** with a coupling agent such as HATU yields the desired isoindoline

Generally speaking, the sequence of steps used to synthesize the compound of formula (I) and further functionalization can also be modified in certain cases.

Insofar as its preparation is not described in the examples, the compound of formula (I) as well as all intermediate products can be prepared according to analogous methods or according to the methods set forth herein. Starting materials are commercially available, known in the art or can be prepared by methods known in the art or in analogy thereto.

It will be appreciated that the compound of general formula (I) in this invention may be derivatised at functional groups to provide derivatives which are capable of conversion back to the parent compound *in vivo.*

A certain embodiment of the invention relates to the compound of formula (I) as described herein, or a pharmaceutically acceptable salt thereof, for use as therapeutically active substance.

A certain embodiment of the invention relates to the compound of formula (I) as described herein, or a pharmaceutically acceptable salt thereof, for the use in the therapeutic and/or prophylactic treatment of cancer, in particular non-small-cell lung cancer.

A certain embodiment of the invention relates to the compound of formula (I) as described herein, or a pharmaceutically acceptable salt thereof, for the use in the therapeutic and/or prophylactic treatment of non-small-cell lung cancer.

A certain embodiment of the invention relates to the compound of formula (I) as described herein, or a pharmaceutically acceptable salt thereof, for the manufacture of a medicament for the therapeutic and/or prophylactic treatment of cancer, in particular non-small-cell lung cancer.

A certain embodiment of the invention relates to a pharmaceutical composition comprising the compound of formula (I) as described herein, or a pharmaceutically acceptable salt thereof, and a pharmaceutically acceptable auxiliary substance.

Disclosed but not claimed is a method for the therapeutic and/or prophylactic treatment of cancer, in particular non-small-cell lung cancer by administering the compound of formula (I) as described herein, or a pharmaceutically acceptable salt thereof, to a patient in need thereof.

A certain embodiment of the invention relates to the compound of formula (I) as described herein, or a pharmaceutically acceptable salt thereof, for the use as a medicament in therapeutic and/or prophylactic treatment of a patient with EGFR activating mutations suffering from cancer, in particular non-small-cell lung cancer, comprising determining the EGFR activating mutations status in said patient and then administering the compound of formula (I) as described herein, or a pharmaceutically acceptable salt thereof, to said patient.

A certain embodiment of the invention relates to the compound of formula (I) as described herein, or a pharmaceutically acceptable salt thereof, for the use as a medicament in therapeutic and/or prophylactic treatment of a patient with EGFR mutations T790M/L858R, T790M/L858R/C797S, L858R and/or L858R/C797S suffering from cancer, in particular non-small-cell lung cancer, comprising determining the EGFR activating mutations status in said patient and then administering the compound of formula (I) as described herein, or a pharmaceutically acceptable salt thereof, to said patient.

A certain embodiment of the invention relates to the compound of formula (I) as described herein, or a pharmaceutically acceptable salt thereof, for the use as a medicament in therapeutic and/or prophylactic treatment of a patient with EGFR activating mutations as determined with a cobas^{®} EGFR Mutation Test v2 suffering from cancer, in particular non-small-cell lung cancer, comprising determining the EGFR activating mutations status in said patient and then administering the compound of formula (I) as described herein, or a pharmaceutically acceptable salt thereof, to said patient.

The invention also relates in particular to:
A compound of formula (I) for use as therapeutically active substance;
A pharmaceutical composition comprising a compound of formula (I) and a therapeutically inert carrier;
A compound of formula (I) for use in the treatment or prophylaxis of cancer;
A compound of formula (I) for use in the treatment or prophylaxis of non-small cell lung cancer;
Disclosed but not claimed is the use of a compound of formula (I) for the treatment or prophylaxis of cancer;
Disclosed but not claimed is the use of a compound of formula (I) for the treatment or prophylaxis of cancer for the treatment or prophylaxis of non-small cell lung cancer;
The use of a compound of formula (I) for the preparation of a medicament for the treatment or prophylaxis of cancer;
The use of a compound of formula (I) for the preparation of a medicament for the treatment or prophylaxis of non-small cell lung cancer;
Disclosed but not claimed is a method for the treatment or prophylaxis of cancer, which method comprises administering an effective amount of a compound of formula (I) to a patient in need thereof; and
Disclosed but not claimed is a Or method for the treatment or prophylaxis of non-small cell lung cancer, which method comprises administering an effective amount of a compound of formula (I) to a patient in need thereof.

Furthermore, the invention includes all substituents in its corresponding deuterated form, wherever applicable, of the compound of formula (I).

Furthermore, the invention includes all optical isomers, i.e. diastereoisomers, diastereomeric mixtures, racemic mixtures, all their corresponding enantiomers and/or tautomers as well as their solvates, wherever applicable, of the compound of formula (I).

The compound of formula (I) may contain one or more asymmetric centers and can therefore occur as racemates, racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compound are included within this invention. The present invention is meant to encompass all such isomeric forms of these compound. The independent syntheses of these diastereomers or their chromatographic separations may be achieved as known in the art by appropriate modification of the methodology disclosed herein. Their absolute stereochemistry may be determined by the x-ray crystallography of crystalline products or crystalline intermediates which are derivatized, if necessary, with a reagent containing an asymmetric center of known absolute configuration. If desired, racemic mixtures of the compound may be separated so that the individual enantiomers are isolated. The separation can be carried out by methods well known in the art, such as the coupling of a racemic mixture of compound to an enantiomerically pure compound to form a diastereomeric mixture, followed by separation of the individual diastereomers by standard methods, such as fractional crystallization or chromatography.

In the embodiments, where optically pure enantiomers are provided, optically pure enantiomer means that the compound contains > 90 % of the desired isomer by weight, particularly > 95 % of the desired isomer by weight, or more particularly > 99 % of the desired isomer by weight, said weight percent based upon the total weight of the isomer(s) of the compound. Chirally pure or chirally enriched compound may be prepared by chirally selective synthesis or by separation of enantiomers. The separation of enantiomers may be carried out on the final product or alternatively on a suitable intermediate.

Also an embodiment of the present invention are compound of formula (I) as described herein, when manufactured according to any one of the described processes.

### Assay procedures

The compound of formula (I) and their pharmaceutically acceptable salts possess valuable pharmacological properties. The compound was investigated in accordance with the test given hereinafter.

### HTRF Phospho EGFR TMLRCS assay (cellular)

### Cell line and media

BaF3-TMLRCS cell line were obtained from Crownbio (San Diego, CA, USA). Cells were maintained at 37°C, 5% CO₂ in RPMI ATCC (Gibco 31870) + 2mM Glutamine + 0.5µg/ml Puromycin supplemented with 10% fetal bovine serum (FBS) (Gibco).

### Protocol

Cells are transferred as above to Greiner Bio-One, Nr. 784-08 micro-titerplate at 20000 cells/well in 12.5 µl of growth medium/well after the plates had been pre-filled with 12.5 nl of DMSO solutions of the to be tested compound (in dose response) or DMSO only. After spinning the plates at 300 x g for 30 seconds the cells were incubated for 4 hours at 37C, 5% CO2, 95% humidity. The cells were lysed by adding to the compound mix 4 µl/well of the supplemented lysis buffer (Cis-bio, Phospho-EGFR HTRF kit, 64EG1PEH), followed by incubation for 30 min at room temperature with shaking (400 rpm). The plates were then frozen and stored overnight at - 80C. On the next day and after thawing the plates, 4 µl of a mixture of anti-Phospho-EGFR Cryptate and of anti-Phospho-EGFR-d2 antibody solutions prepared in the supplied detection buffer was added to each well. The lidded plates were then incubated for 4 h at room temperature before reading the fluorescence emission at 616 and 665 nm using an Envision reader (Perkin Elmer). Data was analyzed in similar fashion as above using the normalized ratio of the 665 to 616 signals multiplied by 10000.

The results are shown in Table 1.

**Table 1: BaF3 cellular HTRF Phospho EGFR TMLRCS assay data**

| **Exam.** | **Structure** | **IC₅₀** (BaF3 TMLRCS) |
|---|---|---|
| 1 | 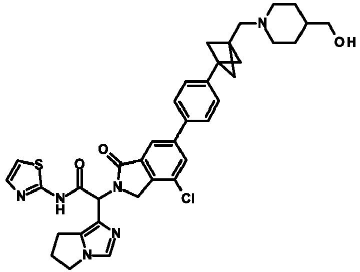 | 9nM |
| 2 | 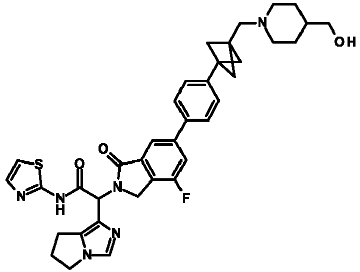 | 4nM |
| 3 | 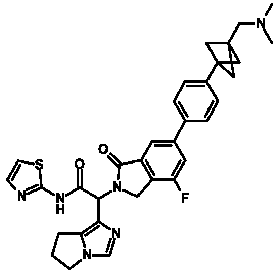 | 10nM |
| 4 | 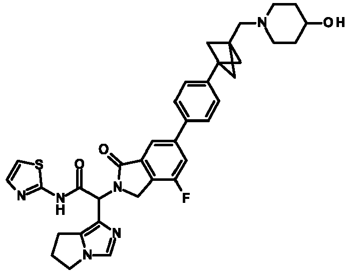 | 13nM |
| 5 | 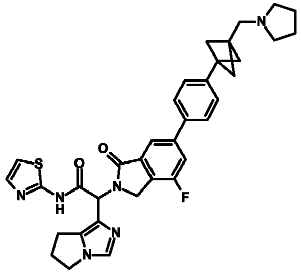 | 13nM |
| 6 | 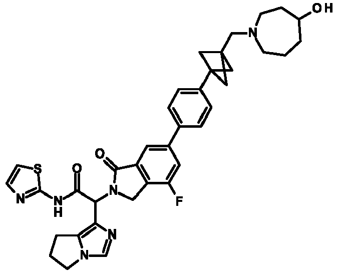 | 8nM |
| 7 | 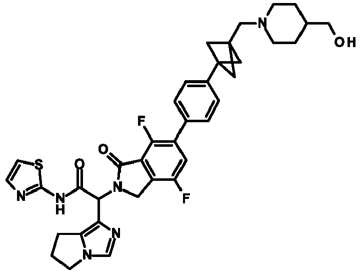 | 4nM |

The compound of formula (I) and their pharmaceutically acceptable salts can be used as medicaments (e.g. in the form of pharmaceutical preparations). The pharmaceutical preparations can be administered internally, such as orally (e.g. in the form of tablets, coated tablets, dragées, hard and soft gelatin capsules, solutions, emulsions or suspensions), nasally (e.g. in the form of nasal sprays), rectally (e.g. in the form of suppositories) or topical ocularly (e.g. in the form of solutions, ointments, gels or water soluble polymeric inserts). However, the administration can also be effected parenterally, such as intramuscularly, intravenously, or intraocularly (e.g. in the form of sterile injection solutions).

The compound of formula (I) and their pharmaceutically acceptable salts can be processed with pharmaceutically inert, inorganic or organic adjuvants for the production of tablets, coated tablets, dragées, hard gelatin capsules, injection solutions or topical formulations Lactose, corn starch or derivatives thereof, talc, stearic acid or its salts etc. can be used, for example, as such adjuvants for tablets, dragées and hard gelatin capsules.

Suitable adjuvants for soft gelatin capsules, are, for example, vegetable oils, waxes, fats, semi-solid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semi-solid or liquid polyols, etc.

Suitable adjuvants for topical ocular formulations are, for example, cyclodextrins, mannitol or many other carriers and excipients known in the art.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should it be appropriate. In the case of topical administration, the formulation can contain 0.001% to 15% by weight of medicament and the required dose, which can be between 0.1 and 25 mg in can be administered either by single dose per day or per week, or by multiple doses (2 to 4) per day, or by multiple doses per week It will, however, be clear that the upper or lower limit given herein can be exceeded when this is shown to be indicated.

### Pharmaceutical Compositions

The compound of formula (I) and the pharmaceutically acceptable salts can be used as therapeutically active substances, e.g. in the form of pharmaceutical preparations. The pharmaceutical preparations can be administered orally, e.g. in the form of tablets, coated tablets, dragées, hard and soft gelatin capsules, solutions, emulsions or suspensions. The administration can, however, also be effected rectally, e.g. in the form of suppositories, or parenterally, e.g. in the form of injection solutions.

The compound of formula (I) and the pharmaceutically acceptable salts thereof can be processed with pharmaceutically inert, inorganic or organic carriers for the production of pharmaceutical preparations. Lactose, corn starch or derivatives thereof, talc, stearic acids or its salts and the like can be used, for example, as such carriers for tablets, coated tablets, dragées and hard gelatin capsules. Suitable carriers for soft gelatin capsules are, for example, vegetable oils, waxes, fats, semi-solid and liquid polyols and the like. Depending on the nature of the active substance no carriers are however usually required in the case of soft gelatin capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical preparations can, moreover, contain pharmaceutically acceptable auxiliary substances such as preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Medicaments containing a compound of formula (I) or a pharmaceutically acceptable salt thereof and a therapeutically inert carrier are also provided by the present invention, as is a process for their production, which comprises bringing one or more compound of formula (I) and/or pharmaceutically acceptable salts thereof and, if desired, one or more other therapeutically valuable substances into a galenical administration form together with one or more therapeutically inert carriers.

The dosage can vary within wide limits and will, of course, have to be adjusted to the individual requirements in each particular case. In the case of oral administration the dosage for adults can vary from about 0.01 mg to about 1000 mg per day of a compound of general formula (I) or of the corresponding amount of a pharmaceutically acceptable salt thereof. The daily dosage may be administered as single dose or in divided doses and, in addition, the upper limit can also be exceeded when this is found to be indicated.

The following examples illustrate the present invention without limiting it, but serve merely as representative thereof. The pharmaceutical preparations conveniently contain about 1-500 mg, particularly 1-100 mg, of a compound of formula (I). Examples of compositions according to the invention are:

### Example A

Tablets of the following composition are manufactured in the usual manner:

**Table 2: possible tablet composition**

| ingredient | mg/tablet | | | |
|---|---|---|---|---|
| | 5 | 25 | 100 | 500 |
| Compound of formula (I) | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

### Example B-1

Capsules of the following composition are manufactured:

**Table 3: possible capsule ingredient composition**

| ingredient | mg/capsule | | | |
|---|---|---|---|---|
| | 5 | 25 | 100 | 500 |
| Compound of formula (I) | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

The compound of formula (I), lactose and corn starch are firstly mixed in a mixer and then in a comminuting machine. The mixture is returned to the mixer; the talc is added thereto and mixed thoroughly. The mixture is filled by machine into suitable capsules, e.g. hard gelatin capsules.

### Example B-2

Soft Gelatin Capsules of the following composition are manufactured:

**Table 4: possible soft gelatin capsule ingredient composition**

| ingredient | mg/capsule |
|---|---|
| Compound of formula (I) | 5 |
| Yellow wax | 8 |
| Hydrogenated Soya bean oil | 8 |
| Partially hydrogenated plant oils | 34 |
| Soya bean oil | 110 |
| Total | 165 |

**Table 5: possible soft gelatin capsule composition**

| ingredient | mg/capsule |
|---|---|
| Gelatin | 75 |
| Glycerol 85 % | 32 |
| Karion 83 | 8 (dry matter) |
| Titan dioxide | 0.4 |
| Iron oxide yellow | 1.1 |
| Total | 116.5 |

### Manufacturing Procedure

The compound of formula (I) is dissolved in a warm melting of the other ingredients and the mixture is filled into soft gelatin capsules of appropriate size. The filled soft gelatin capsules are treated according to the usual procedures.

### Example C

Suppositories of the following composition are manufactured:

**Table 6: possible suppository composition**

| ingredient | mg/supp. |
|---|---|
| Compound of formula (I) | 15 |
| Suppository mass | 1285 |
| Total | 1300 |

### Manufacturing Procedure

The suppository mass is melted in a glass or steel vessel, mixed thoroughly and cooled to 45°C. Thereupon, the finely powdered compound of formula (I) is added thereto and stirred until it has dispersed completely. The mixture is poured into suppository moulds of suitable size, left to cool; the suppositories are then removed from the moulds and packed individually in wax paper or metal foil.

### Example D

Injection solutions of the following composition are manufactured:

**Table 7: possible injection solution composition**

| ingredient | mg/injection solution. |
|---|---|
| Compound of formula (I) | 3 |
| Polyethylene Glycol 400 | 150 |
| acetic acid | q.s. ad pH 5.0 |
| water for injection solutions | ad 1.0 ml |

### Manufacturing Procedure

The compound of formula (I) is dissolved in a mixture of Polyethylene Glycol 400 and water for injection (part). The pH is adjusted to 5.0 by acetic acid. The volume is adjusted to 1.0 ml by addition of the residual amount of water. The solution is filtered, filled into vials using an appropriate overage and sterilized.

### Example E

Sachets of the following composition are manufactured:

**Table 8: possible sachet composition**

| ingredient | mg/sachet |
|---|---|
| Compound of formula (I) | 50 |
| Lactose, fine powder | 1015 |
| Microcrystalline cellulose (AVICEL PH 102) | 1400 |
| Sodium carboxymethyl cellulose | 14 |
| Polyvinylpyrrolidon K 30 | 10 |
| Magnesium stearate | 10 |
| Flavoring additives | 1 |
| Total | 2500 |

### Manufacturing Procedure

The compound of formula (I) is mixed with lactose, microcrystalline cellulose and sodium carboxymethyl cellulose and granulated with a mixture of polyvinylpyrrolidone in water. The granulate is mixed with magnesium stearate and the flavoring additives and filled into sachets.

### Examples

The following examples are provided for illustration of the invention. They should not be considered as limiting the scope of the invention, but merely as being representative thereof.

### Example 1

### (2RS)-2-(6,7-Dihydro-5H-pyrrolo[1,2-c]imidazol-1-yl)-2-[4-chloro-6-[4-[3-[[4-(hydroxymethyl)-1-piperidyl]methyl]-1-bicyclo[1.1.1]pentanyl]phenyl]-1-oxo-isoindolin-2-yl]-N-thiazol-2-yl-acetamide

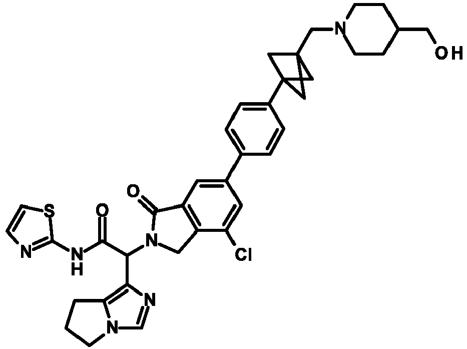

### Step 1: Ethyl 2-(6,7-dihydro-5H-pyrrolo[1,2-c]imidazol-1-yl)-2-oxo-acetate

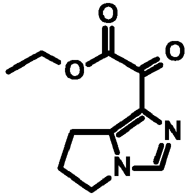

To a solution of ethyl 2-(6,7-dihydro-SH-pyrrolo[1,2-c]imidazol-1-yl)acetate (20.0 g, 102.97 mmol) dissolved in 200 ml of 1,4-dioxane was added selenium dioxide (22.85 g, 205.94 mmol, 2 equiv.). The reaction mixture was stirred for 5 hours at 80°C. The reaction mixture was concentrated under vacuum to give a residue. The crude product was purified by flash chromatography on a silica gel column eluting with petroleum ether:ethyl acetate 2:1 to ethyl acetate:ethanol 10:1 gradient to obtain the desired ethyl 2-(6,7-dihydro-5H-pyrrolo[1,2-c]imidazol-1-yl)-2-oxo-acetate (quant. yield) as a light brown oil, MS: m/e = 209.1 (M+H⁺).

### Step 2: Ethyl 2-(6,7-dihydro-5H-pyrrolo[1,2-c]imidazol-1-yl)-2-hydroxyimino-acetate

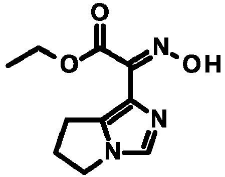

To a solution of ethyl 2-(6,7-dihydro-5H-pyrrolo[1,2-c]imidazol-1-yl)-2-oxo-acetate *(Example 1, step 1)* (17.5 g, 84.05 mmol) dissolved in 145 ml of ethanol was added hydroxylamine hydrochloride (6.42 g, 92.45 mmol, 1.1 equiv.) and sodium acetate (13.79 g, 168.1 mmol, 2 equiv.) at room temperature. The reaction mixture was stirred for 3.5 hours at 80°C. The reaction mixture was concentrated and extracted with water and five times with a mixture of ethanol/THF/ethyl acetate 1:1:8. The organic layers were concentrated to dryness. The desired ethyl 2-(6,7-dihydro-SH-pyrrolo[1,2-c]imidazol-1-yl)-2-hydroxyimino-acetate (15 g, 80 % yield) was obtained as a yellow solid, MS: m/e = 224.1 (M+H⁺) and used directly in the next step.

### Step 3: Ethyl (2RS)-2-amino-2-(6,7-dihydro-5H-pyrrolo[1,2-c]imidazol-1-yl)acetate

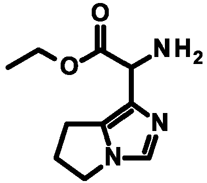

To a solution of ethyl 2-(6,7-dihydro-5H-pyrrolo[1,2-c]imidazol-1-yl)-2-hydroxyimino-acetate *(Example 1*, *step 2)* (15.0 g, 67.2 mmol) dissolved in 225 ml of ethanol and 120 ml of THF was added Pd/C (30.0g, 67.2 mmol, 1 eq, 10%) at room temperature. The mixture was hydogenated with H₂ for 24 hours at 45°C. The reaction mixture was filtered and the filtrate was concentrated under vacuum. The desired ethyl (2*RS*)-2-amino-2-(6,7-dihydro-5H-pyrrolo[1,2-c]imidazol-1-yl)acetate (quant, yield) was obtained as a brown oil, MS: m/e = 210.1 (M+H⁺) and used directly in the next step.

### Step 4: Ethyl (2RS)-2-amino-2-(6,7-dihydro-5H-pyrrolo[1,2-c]imidazol-1-yl)acetate hydrochloride

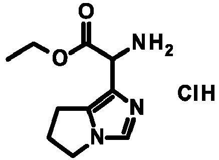

A solution of ethyl (2*RS*)-2-amino-2-(6,7-dihydro-5H-pyrrolo[1,2-c]imidazol-1-yl)acetate *(Example 1, step 3)* (15.0 g, 82.79 mmol) in HCl/EtOH (300 ml, 1200 mmol, 14.5 equiv., 2.5 mol/L) was stirred at 25 °C for 36 hours. The reaction mixture was concentrated under vacuum below 25°C to give a residue as brown oil. 150 ml of acetonitrile were added to the residue and the precipitated yellow solid was collected and dried under vacuum below 25 °C to give the desired ethyl (2*RS*)-2-amino-2-(6,7-dihydro-5H-pyrrolo[1,2-c]imidazol-1-yl)acetate hydrochloride (quant. yield) as yellow solid, MS: m/e = 210.1 (M+H⁺).

### Step 5: Ethyl 2-(bromomethyl)-3-chloro-5-iodo-benzoate

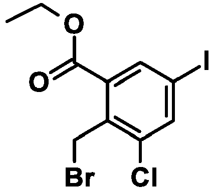

3-Chloro-5-iodo-2-ethyl-benzoate (57.3 g, 176 mmol) was dissolved in 400 ml tetrachloroethylene and N-bromosuccinimide (46.9 g, 265 mmol, 1.5 equiv.) and AIBN (13.4 g, 88.3 mmol, 0.5 equiv.) were added at room temperature. The mixture was stirred at 80°C for 16 hours. The reaction mixture was concentrated to dryness. The crude product was purified by flash chromatography on a silica gel column eluting with a petroleum ether:ethyl acetate 1:0 to 10:1 gradient to obtain the desired product (56 g, 76 % yield) as a pink solid.

### Step 6: Ethyl (2RS)-2-(4-chloro-6-iodo-1-oxo-isoindolin-2-yl)-2-(6,7-dihydro-5H-pyrrolo[1,2-c]imidazol-1-yl)acetate

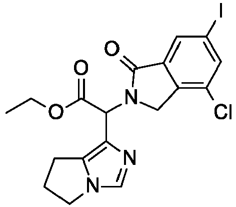

Ethyl (2*RS*)-2-amino-2-(6,7-dihydro-5H-pyrrolo[1,2-c]imidazol-1-yl)acetate hydrochloride *(Example 1, step 4)* (12 g, 48.8 mmol, 1 equiv.) was dissolved in 120 ml of dioxane and 20 ml of DMF. Ethyl 2-(bromomethyl)-3-chloro-5-iodo-benzoate *(Example 1, step 5)* (19.7 g, 48.8 mmol) and diisopropylethylamine (34 ml, 195 mmol, 4 equiv.) were added at room temperature. The mixture was stirred at room temperature for 30 minutes and at 60°C for 2 hours. The reaction mixture was extracted with water and two times with ethyl acetate. The organic layers were extracted with brine, dried over sodium sulfate and concentrated to dryness. The crude product was purified by flash chromatography on a silica gel column eluting with an ethyl acetate:methanol 100:0 to 90: 10 gradient to obtain the desired product (14.5 g, 55 % yield) as a light red solid, MS: m/e = 486.2 (M+H⁺).

### Step 7: [3-(4-Bromophenyl)-1-bicyclo[1.1.1]pentanyl]methanol

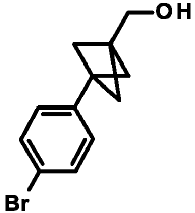

3-(4-Bromophenyl)bicyclo[1.1.1]pentane-1-carboxylic acid *(*CAS 1980054-39-4*)* (500 mg, 1.77 mmol) was dissolved in 4 ml of THF and the mixture was cooled to 0-5 °C. Borane-methyl sulfide complex (2 M in THF) (1.0 ml, 2.04 mmol, 1.15 equiv.) was added drop wise at 0-5 °C and the mixture was stirred for 16 hours at room temperature. 10 ml of methanol were added drop wise and the mixture was concentrated to dryness. The residue was extracted with saturated sodiumcarbonate solution and three times with TBME. The organic layers were extracted with brine, dried over sodium sulfate and concentrated to dryness to obtain the desired product (478 mg, quantitative yield) as a white solid, MS: m/e = 236.9 (M+H⁺).

### Step 8: 3-(4-Bromophenyl)bicyclo[1.1.1]pentane-1-carbaldehyde

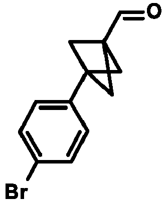

[3-(4-Bromophenyl)-1-bicyclo[1.1.1]pentanyl]methanol *(Example 1*, *step 7)* (478 mg, 1.66 mmol) was dissolved in 11 ml of dichloromethane and the mixture was cooled to 0-5 °C. Dess-Martin periodinane (900 mg, 2.07 mmol, 1.25 equiv.) was added at 0-5 °C and the mixture was stirred for 2 hours at room temperature. 40 ml of diethyl ether were added and the mixture was filtered. The filtrate was extracted with 1M sodium hydroxide solution and three times with diethyl ether. The organic layers were extracted with 5% aqueous sodium thiosulfate solution and brine, dried over sodium sulfate and concentrated to dryness to obtain the desired product (430 mg, quantitative yield) as a white solid.

### Step 9: [1-[[3-(4-Bromophenyl)-1-bicyclo[1.1.1]pentanyl]methyl]-4-piperidyl]methanol

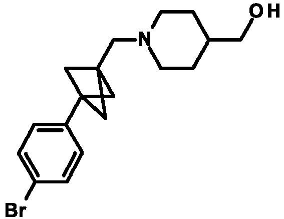

3-(4-Bromophenyl)bicyclo[1.1.1]pentane-1-carbaldehyde *(Example 1, step 8)* (430 mg, 1.63 mmol) was dissolved in 8 ml of dichloromethane. Piperidin-4-ylmethanol (225 mg, 1.95 mmol, 1.2 equiv.) and sodium triacetoxyborohydride (517 mg, 2.44 mmol, 1.5 equiv.) were added at room temperature. The mixture was stirred at room temperature for 3 hours. The reaction mixture was extracted with saturated sodiumhydrogencarbonate solution and three times with dichloromethane. The organic layers were extracted with brine, dried over sodium sulfate and concentrated to dryness. The crude product was purified by flash chromatography on a silica gel column eluting with a dichloromethane:methanol+1% ammonia 100:0 to 90:10 gradient to obtain the desired product (495 mg, 70 % yield) as a white solid, MS: m/e = 352.0 (M+H⁺).

### Step 10: [1-[[3-[4-(4,4,5,5-Tetramethyl-1,3,2-dioxaborolan-2-yl)phenyl]-1-bicyclo[1.1.1.]pentanyl]methyl]-4-piperidyl]methanol

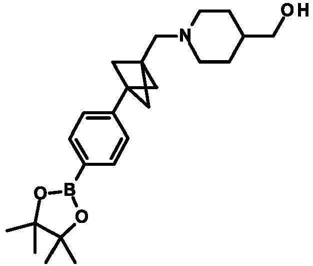

[1-[[3-(4-Bromophenyl)-1-bicyclo[1.1.1]pentanyl]methyl]-4-piperidyl]methanol *(Example 1, step 9)* (295 mg, 0.84 mmol) and bis(pinacolato)diboron (235 mg, 0.93 mmol, 1.1 equiv.) were dissolved in 7 ml of dioxane. Potassium acetate (248 mg, 2.53 mmol, 3.0 equiv.) and dichloro 1,1'-bis(diphenylphosphino)ferrocene palladium(II) dichloromethane adduct (75 mg, 0.09 mmol, 0.11 equiv.) were added and the reaction mixture was stirred at 90°C for 40 minutes. The reaction mixture was cooled to room temperature and concentrated to dryness. The crude product was purified by flash chromatography on an amino-silica gel column eluting with a dichloromethane:methanol 100:0 to 90:10 gradient. The desired product (286 mg, 79 % yield) was obtained as a brown solid, MS: m/e = 398.3 (M+H⁺).

### Step 11: Ethyl (2RS)-2-[4-chloro-6-[4-[3-[[4-(hydroxymethyl)-1-piperidyl]methyl]-1-bicyclo[1.1.1]pentanyl]phenyl]-1-oxo-isoindolin-2-yl]-2-(6,7-dihydro-5H-pyrrolo[1,2-c]imidazol-1-yl)acetate

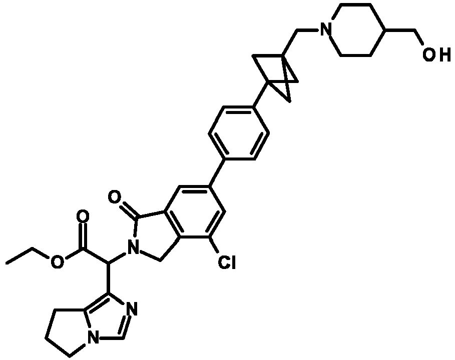

Ethyl (2RS)-2-(4-chloro-6-iodo-1-oxo-isoindolin-2-yl)-2-(6,7-dihydro-5H-pyrrolo[1,2-c]imidazol-1-yl)acetate *(Example 1*, *step 6)* (70 mg, 0.18 mmol) and [1-[[3-[4-(4,4,5,5-tetramethyl-1,3,2-dioxaborolan-2-yl)phenyl]-1-bicyclo[1.1.1]pentanyl]methyl]-4-piperidyl]methanol *(Example 1*, *step 10)* (86 mg, 0.18 mmol, 1.0 equiv.) were dissolved in 1.5 ml of THF and 0.25 ml of water. Sodium carbonate (56 mg, 0.53 mmol, 3.0 equiv.) and PdCl2(DPPF)-CH2Cl2 adduct (26 mg, 0.035 mmol, 0.2 equiv.) were added and the reaction mixture was stirred at 70°C for 6 hours. The reaction mixture was cooled to room temperature and then extracted with ethyl acetate and water. The aqueous layer was back-extracted twice with ethyl acetate. The organic layers were washed with brine. The organic layers were combined, dried over sodium sulfate, filtered and concentrated to dryness. The crude product was purified by flash chromatography on a silica gel column eluting with a dichloromethane:methanol 100:0 to 80:20 gradient. The desired product (53 mg, 44 % yield) was obtained as a brown solid, MS: m/e = 629.3 (M+H⁺).

### Step 12: (2RS)-2-(6,7-Dihydro-5H-pyrrolo[1,2-c]imidazol-1-yl)-2-[4-chloro-6-[4-[3-[[4-(hydroxymethyl)-1-piperidyl]methyl]-1-bicyclo[1.1.1]pentanyl]phenyl]-1-oxo-isoindolin-2-yl]-N-thiazol-2-yl-acetamide

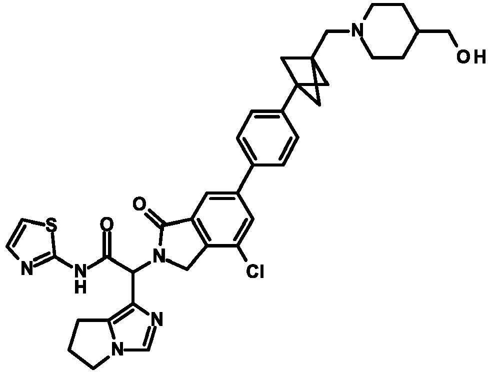

Ethyl (2RS)-2-[4-chloro-6-[4-[3-[[4-(hydroxymethyl)-1-piperidyl]methyl]-1-bicyclo[1.1.1]pentanyl]phenyl]-1-oxo-isoindolin-2-yl]-2-(6,7-dihydro-5H-pyrrolo[1,2-c]imidazol-1-yl)acetate *(Example 1*, *step 11)* (53 mg, 0.083 mmol) was dissolved in 2 ml of ethanol. LiOH (1M in water) (0.12 ml, 0.12 mmol, 1.5 equiv.) was added at room temperature. The mixture was stirred for 16 hours at room temperature. The reaction mixture was concentrated in vacuo to dryness and the residue was dissolved in 1 ml of DMF. Thiazol-2-amine (9 mg, 0.091 mmol, 1.1 equiv.), Hunig's base (0.058 ml, 0.334 mmol, 4 equiv.) and HATU (48 mg, 0.125 mmol, 1.5 equiv.) were added at room temperature. The mixture was stirred at room temperature for 16 hours. The reaction mixture was extracted with water and three times with ethyl acetate. The organic layers were extracted with water, dried over sodium sulfate and concentrated to dryness. The crude product was purified by flash chromatography on an amine-modified silica gel column eluting with a ethyl acetate: methanol 100:0 to 80:20 gradient to obtain the desired product (20 mg, 35 % yield) as a brown solid, MS: m/e = 683.3 (M+H⁺).

### Example 2

### (2RS)-2-(6,7-Dihydro-SH-pyrrolo [1,2-c] imidazol-1-yl)-2-[4-fluoro-6-[4-[3-[[4-(hydroxymethyl)-1-piperidyl]methyl]-1-bicyclo[1.1.1]pentanyl]phenyl]-1-oxo-isoindolin-2-yl]-N-thiazol-2-yl-acetamide

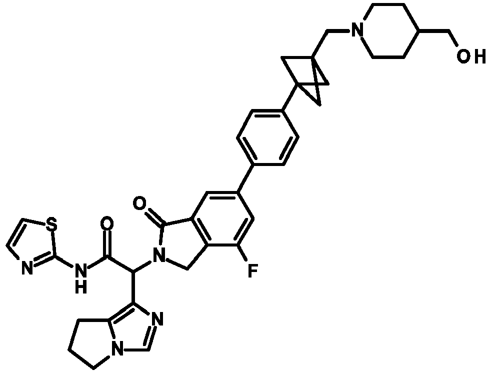

### Step 1: Methyl 5-bromo-2-(bromomethyl)-3-fluoro-benzoate

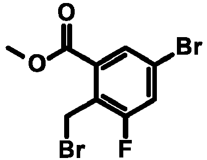

Methyl 5-bromo-3-fluoro-2-methylbenzoate (CAS 2090424-20-5, 5.91 g, 23.9 mmol) was dissolved in 100 ml trifluorotoluene and N-bromosuccinimide (4.26 g, 23.9 mmol, 1 equiv.) and AIBN (393 mg, 2.39 mmol, 0.1 equiv.) were added at room temperature. The mixture was stirred at 110°C for 3 hours. The reaction mixture was cooled, extracted with water and two times with ethyl acetate. The organic layers were dried over sodium sulfate and concentrated to dryness. The crude product was purified by flash chromatography on a silica gel column eluting with an ethyl acetate:heptane 0: 100 to 50:50 gradient to obtain the desired methyl 5-bromo-2-(bromomethyl)-3-fluoro-benzoate (7.29 g, 94 % yield) as a light yellow liquid, MS: m/e = 326.8 (M+H⁺).

### Step 2: Ethyl (2RS)-2-(6-bromo-4-fluoro-1-oxo-isoindolin-2-yl)-2-(6,7-dihydro-5H-pyrrolo[1,2-c]imidazol-1-yl)acetate

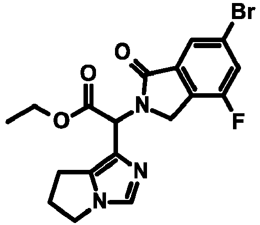

Ethyl (2*RS*)-2-amino-2-(6,7-dihydro-5H-pyrrolo[1,2-c]imidazol-1-yl)acetate hydrochloride *(Example 1, step 4)* (4.15 g, 16.9 mmol, 1 equiv.) was dissolved in 35 ml of DMF. Methyl 5-bromo-2-(bromomethyl)-3-fluoro-benzoate *(Example 2*, *step 1)* (5.0 g, 15.3mmol) and triethylamine (10.7 ml, 76.7 mmol, 5 equiv.) were added at room temperature. The mixture was stirred at 80°C for 16 hours. The reaction mixture was extracted with water and two times with ethyl acetate. The organic layers were extracted with brine, dried over sodium sulfate and concentrated to dryness. The crude product was purified by flash chromatography on a silica gel column eluting with a dichloromethane:methanol 100:0 to 90:10 gradient to obtain the desired ethyl (2*RS*)-2-(6-bromo-4-fluoro-1-oxo-isoindolin-2-yl)-2-(6,7-dihydro-5H-pyrrolo[1,2-c]imidazol-1-yl)acetate (2.6 g, 40 % yield) as a yellow solid, MS: m/e = 422.1/424.1 (M+H⁺).

### Step 3: [2-[(1RS)-1-(6,7-Dihydro-5H-pyrrolor[1,2-c]imidazol-1-yl)-2-ethoxy-2-oxo-ethyl]-7-fluoro-3-oxo-isoindolin-5-yllboronic acid

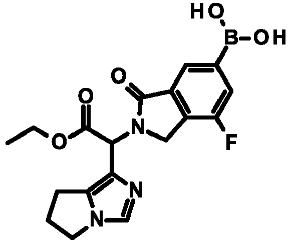

The title compound was obtained as a white solid using chemistry similar to that described in Example 1, step 10 starting from ethyl (2RS)-2-(6-bromo-4-fluoro-1-oxo-isoindolin-2-yl)-2-(6,7-dihydro-5H-pyrrolo[1,2-c]imidazol-1-yl)acetate *(Example 2*, *step 2).*

### Step 4: Ethyl (2RS)-2-[4-fluoro-6-[4-[3-[[4-(hydroxymethyl)-1-piperidyl]methyl]-1-bicyclo[1.1.1]pentanyl]phenyl]-1-oxo-isoindolin-2-yl]-2-(6,7-dihydro-5H-pyrrolo[1,2-c]imidazol-1-yl)acetate

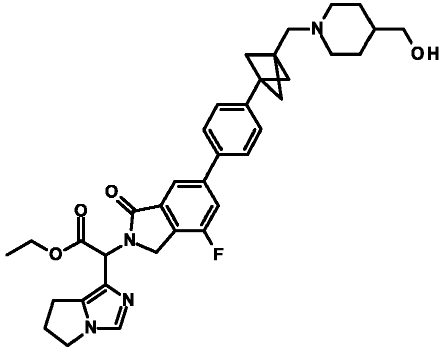

The title compound was obtained as a brown solid, MS: m/e = 613.5 (M+H⁺), using chemistry similar to that described in Example 1, step 11 starting from [2-[(1RS)-1-(6,7-dihydro-5H-pyrrolo[1,2-c]imidazol-1-yl)-2-ethoxy-2-oxo-ethyl]-7-fluoro-3-oxo-isoindolin-5-yl]boronic acid *(Example 2*, *step 3)* and [1-[[3-(4-bromophenyl)-1-bicyclo[1.1.1]pentanyl]methyl]-4-piperidyl]methanol *(Example 1*, *step 9).*

### Step 5: (2RS)-2-(6,7-Dihydro-5H-pyrrolo[1,2-c]imidazol-1-yl)-2-[4-fluoro-6-[4-[3-[[4-(hydroxymethyl)-1-piperidyl]methyl]-1-bicyclo[1.1.1]pentanyl]phenyl]-1-oxo-isoindolin-2-yl]-N-thiazol-2-yl-acetamide

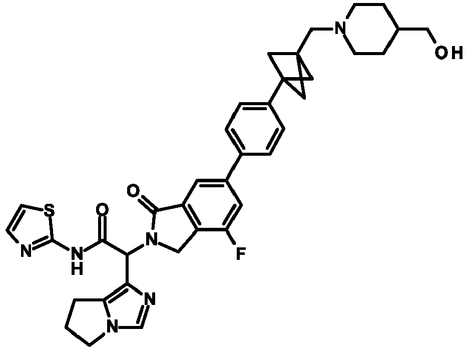

The title compound was obtained as a light brown solid, MS: m/e = 667.4 (M+H⁺), using chemistry similar to that described in Example 1, step 12 starting from ethyl (2RS)-2-[4-fluoro-6-[4-[3-[[4-(hydroxymethyl)-1-piperidyl]methyl]-1-bicyclo[1.1.1]pentanyl]phenyl]-1-oxo-isoindolin-2-yl]-2-(6,7-dihydro-SH-pyrrolo[1,2-c]imidazol-1-yl)acetate *(Example 2*, *step 4)* and thiazol-2-amine.

### Example 3

### (2RS)-2-(6,7-Dihydro-SH-pyrrolo [1,2-c] imidazol-1-yl)-2-[6-[4-[3-[(dimethylamino)methyl]-1-bicyclo[1.1.1]pentanyl]phenyl]-4-fluoro-1-oxo-isoindolin-2-yl]-N-thiazol-2-yl-acetamide

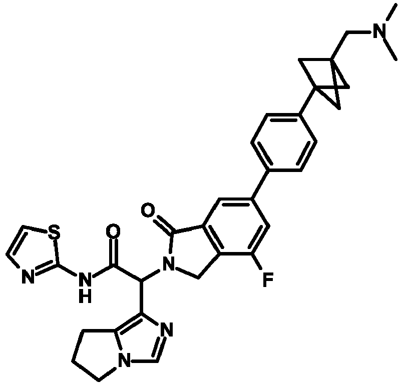

### Step 1: 1-(4-Bromophenyl)-3-(diethoxymethyl)bicyclo[1.1.1]pentane

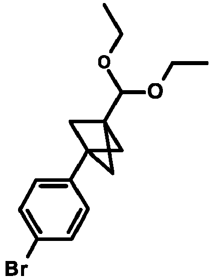

3-(4-Bromophenyl)bicyclo[1.1.1]pentane-1-carbaldehyde *(Example 1*, *step 8)* (168 mg, 0.67 mmol) was dissolved in 7 ml of toluene. Triethyl orthoformate (0.12 ml, 0.74 mmol, 1.1 equiv.) and p-toluenesulfonic acid monohydrate (3 mg, 0.02 mmol, 0.03 equiv.) were added at room temperature. The mixture was stirred at room temperature for 30 minutes. The reaction mixture was extracted with saturated sodiumhydrogencarbonate solution and three times with dichloromethane. The organic layers were extracted with brine, dried over sodium sulfate and concentrated to dryness to obtain the desired product (204 mg, 73 % yield) as a light yellow oil.

### Step 2: Ethyl (2RS)-2-[6-[4-[3-(diethoxymethyl)-1-bicyclo[1.1.1]pentanyl]phenyl]-4-fluoro-1-oxo-isoindolin-2-yll-2-(6,7-dihydro-5H-pyrrolo[1,2-c]imidazol-1-yl)acetate

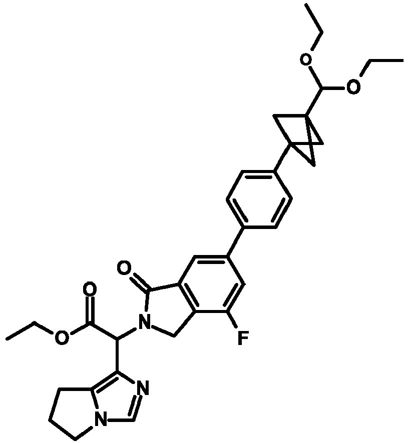

The title compound was obtained as a brown solid, MS: m/e = 588.4 (M+H⁺), using chemistry similar to that described in Example 1, step 11 starting from [2-[(1RS)-1-(6,7-dihydro-5H-pyrrolo[1,2-c]imidazol-1-yl)-2-ethoxy-2-oxo-ethyl]-7-fluoro-3-oxo-isoindolin-5-yl]boronic acid *(Example 2*, *step 3)* and 1-(4-bromophenyl)-3-(diethoxymethyl)bicyclo[1.1.1]pentane *(Example 3, step 1).*

### Step 3: (2RS)-2-[6-[4-[3-(Diethoxymethyl)-1-bicyclo[1.1.1]pentanyl]phenyl]-4-fluoro-1-oxo-isoindolin-2-yl]-2-(6,7-dihydro-5H-pyrrolo[1,2-c]imidazol-1-yl)-N-thiazol-2-yl-acetamide

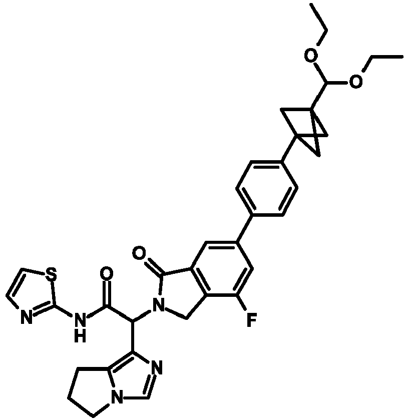

The title compound was obtained as a light brown solid, MS: m/e = 642.4 (M+H⁺), using chemistry similar to that described in Example 1, step 12 starting from ethyl (2RS)-2-[6-[4-[3-(diethoxymethyl)-1-bicyclo[1.1.1]pentanyl]phenyl]-4-fluoro-1-oxo-isoindolin-2-yl]-2-(6,7-dihydro-5H-pyrrolo[1,2-c]imidazol-1-yl)acetate *(Example 3*, *step 2)* and thiazol-2-amine.

### Step 4: (2RS)-2-(6,7-Dihydro-5H-pyrrolo[1,2-c]imidazol-1-yl)-2-[4-fluoro-6-[4-(3-formyl-1-bicyclo[1.1.1]pentanyl)phenyl]-1-oxo-isoindolin-2-yl]-N-thiazol-2-yl-acetamide

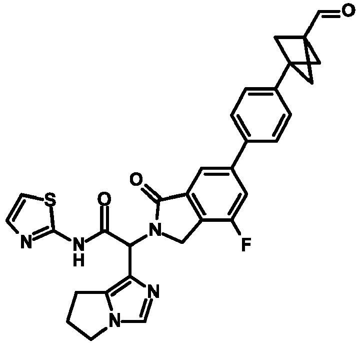

(2RS)-2-[6-[4-[3-(Diethoxymethyl)-1-bicyclo[1.1.1]pentanyl]phenyl]-4-fluoro-1-oxo-isoindolin-2-yl]-2-(6,7-dihydro-5H-pyrrolo[1,2-c]imidazol-1-yl)-N-thiazol-2-yl-acetamide *(Example 3, step 3)* (85 mg, 0.13 mmol) was dissolved in 5 ml of acetone and HCl (37% in water) (0.013 ml, 0.013 mmol, 0.1 equiv.) was added at room temperature. The reaction mixture was stirred at room temperature for 16 hours. The reaction mixture was extracted with ethyl acetate and saturated NaHCO₃-solution. The aqueous layer was backextracted with ethyl acetate. The organic layers were washed with water and brine. The organic layers were combined, dried over sodium sulfate, filtered and concentrated to dryness to obtain the desired product (quantitative yield) as a light brown solid, MS: m/e = 568.2 (M+H⁺).

### Step 5: (2RS)-2-(6,7-Dihydro-5H-pyrrolo[1,2-c]imidazol-1-yl)-2-[6-[4-[3-[(dimethylamino)methyl]-1-bicyclo[1.1.1]pentanyl]phenyl]-4-fluoro-1-oxo-isoindolin-2-yl]-N-thiazol-2-yl-acetamide

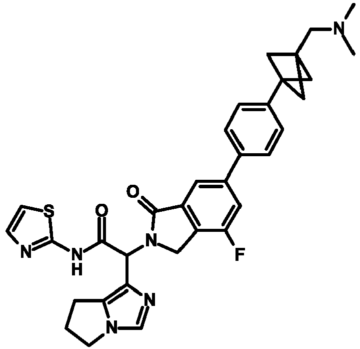

The title compound was obtained as a white solid, MS: m/e = 597.5 (M+H⁺), using chemistry similar to that described in Example 1, step 9 starting from (2RS)-2-(6,7-dihydro-5H-pyrrolo[1,2-c]imidazol-1-yl)-2-[4-fluoro-6-[4-(3-formyl-1-bicyclo[1.1.1]pentanyl)phenyl]-1-oxo-isoindolin-2-yl]-N-thiazol-2-yl-acetamide *(Example 3*, *step 4)* and dimethylamine.

### Example 4

### (2RS)-2-(6,7-Dihydro-SH-pyrrolo [1,2-c] imidazol-1-yl)-2-[4-fluoro-6-[4-[3-[(4-hydroxy-1-piperidyl)methyl]-1-bicyclo[1.1.1]pentanyl]phenyl]-1-oxo-isoindolin-2-yl]-N-thiazol-2-yl-acetamide

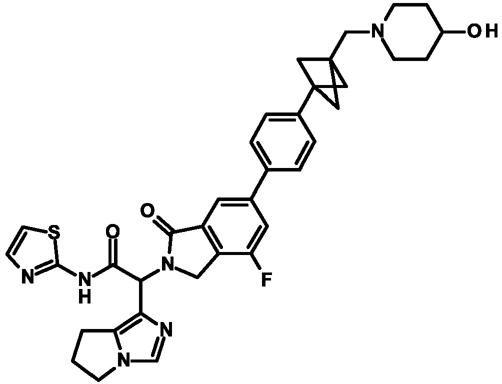

### Step 1: Ethyl (2RS)-2-(6,7-dihydro-5H-pyrrolo[1,2-c]imidazol-1-yl)-2-[4-fluoro-6-[4-(3-formyl-1-bicyclo[1.1.1]pentanyl)phenyl]-1-oxo-isoindolin-2-yl]acetate

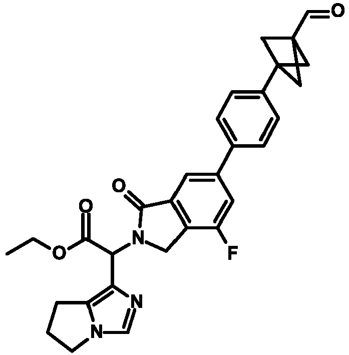

The title compound was obtained as an orange oil, MS: m/e = 514.3 (M+H+), using chemistry similar to that described in Example 1, step 11 starting from [2-[(1RS)-1-(6,7-dihydro-5H-pyrrolo[1,2-c]imidazol-1-yl)-2-ethoxy-2-oxo-ethyl]-7-fluoro-3-oxo-isoindolin-5-yl]boronic acid *(Example 2*, *step 3)* and 3-(4-bromophenyl)bicyclo[1.1.1]pentane-1-carbaldehyde *(Example 1, step 3).*

### Step 2: Ethyl (2RS)-2-(6,7-dihydro-5H-pyrrolo[1,2-c]imidazol-1-yl)-2-[4-fluoro-6-[4-[3-[(4-hydroxy-1-piperidyl)methyl]-1-bicyclo[1.1.1]pentanyl]phenyl]-1-oxo-isoindolin-2-yl]acetate

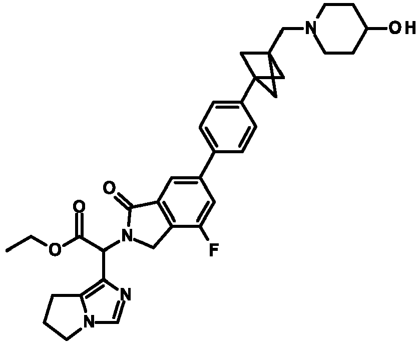

The title compound was obtained as a yellow waxy solid, MS: m/e = 599.4 (M+H⁺), using chemistry similar to that described in Example 1, step 9 starting from ethyl (2RS)-2-(6,7-dihydro-5H-pyrrolo[1,2-c]imidazol-1-yl)-2-[4-fluoro-6-[4-(3-formyl-1-bicyclo[1.1.1]pentanyl)phenyl]-1-oxo-isoindolin-2-yl]acetate *(Example 4*, *step 1)* and piperidin-4-ol.

### Step 3: (2RS)-2-(6,7-Dihydro-5H-pyrrolo[1,2-c]imidazol-1-yl)-2-[4-fluoro-6-[4-[3-[(4-hydroxy-1-piperidyl)methyl]-1-bicyclo[1.1.1]pentanyl]phenyl]-1-oxo-isoindolin-2-yl]-N-thiazol-2-yl-acetamide

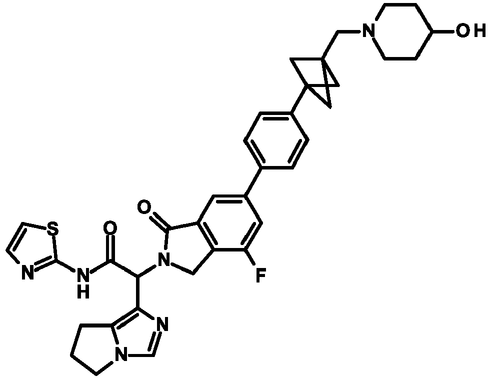

The title compound was obtained as a light brown solid, MS: m/e = 635.5 (M+H⁺), using chemistry similar to that described in Example 1, step 12 starting from ethyl (2RS)-2-(6,7-dihydro-5H-pyrrolo[1,2-c]imidazol-1-yl)-2-[4-fluoro-6-[4-[3-[(4-hydroxy-1-piperidyl)methyl]-1-bicyclo[1.1.1]pentanyl]phenyl]-1-oxo-isoindolin-2-yl]acetate *(Example 4*, *step 2)* and thiazol-2-amine.

### Example 5

### (2RS)-2-(6,7-Dihydro-5H-pyrrolo[1,2-c]imidazol-1-yl)-2-[4-fluoro-1-oxo-6-[4-[3-(pyrrolidin-1-ylmethyl)-1-bicyclo[1.1.1]pentanyl]phenyl]isoindolin-2-yl]-N-thiazol-2-yl-acetamide

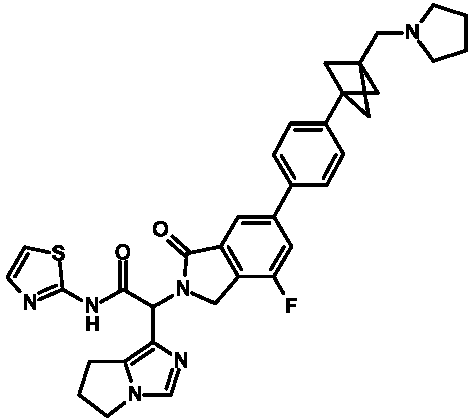

The title compound was obtained as a white solid, MS: m/e = 623.5 (M+H⁺), using chemistry similar to that described in Example 1, step 9 starting from (2RS)-2-(6,7-dihydro-5H-pyrrolo[1,2-c]imidazol-1-yl)-2-[4-fluoro-6-[4-(3-formyl-1-bicyclo[1.1.1]pentanyl)phenyl]-1-oxo-isoindolin-2-yl]-N-thiazol-2-yl-acetamide *(Example 3*, *step 4)* and pyrrolidine.

### Example 6

### (2RS)-2-(6,7-Dihydro-SH-pyrrolo [1,2-c] imidazol-1-yl)-2-[4-fluoro-6-[4-[3-[[(4RS)-4-hydroxyazepan-1-yl]methyl]-1-bicyclo[1.1.1]pentanyl]phenyl]-1-oxo-isoindolin-2-yl]-N-thiazol-2-yl-acetamide

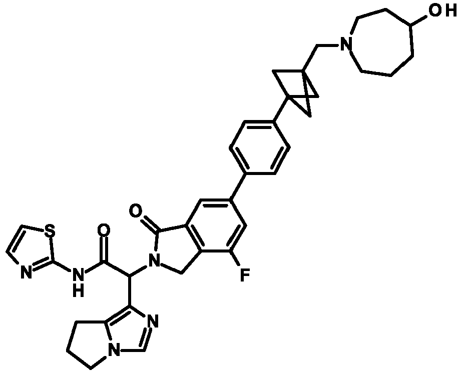

The title compound was obtained as a white solid, MS: m/e = 667.5 (M+H⁺), using chemistry similar to that described in Example 1, step 9 starting from (2RS)-2-(6,7-dihydro-5H-pyrrolo[1,2-c]imidazol-1-yl)-2-[4-fluoro-6-[4-(3-formyl-1-bicyclo[1.1.1]pentanyl)phenyl]-1-oxo-isoindolin-2-yl]-N-thiazol-2-yl-acetamide *(Example 3*, *step 4)* and azepan-4-ol.

### Example 7

### (2RS)-2-[4,7-Difluoro-6-[4-[3-[[4-(hydroxymethyl)-1-piperidyl] methyl]-1-bicyclo[1.1.1]pentanyl]phenyl]-1-oxo-isoindolin-2-yl]-2-(6,7-dihydro-5H-pyrrolo[1,2-c]imidazol-1-yl)-N-thiazol-2-yl-acetamide

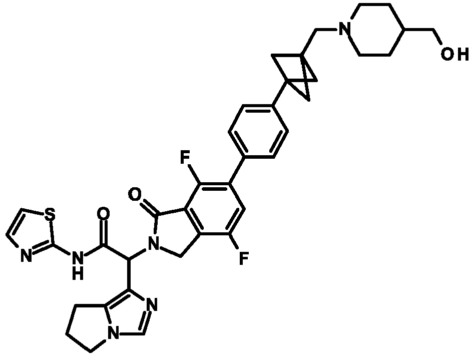

### Step 1: 2-Fluoro-3-iodo-6-methyl-5-nitrobenzoic acid

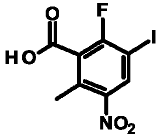

6-Fluoro-2-methyl-3-nitrobenzoic acid (1.28 g, 6.43 mmol) was dissolved in 5 ml of sulfuric acid. N-iodosuccinimide (1.59 g, 7.07 mmol, 1.1 equiv.) was added at room temperature. The mixture was stirred at room temperature for 16 hours. The reaction mixture was poured onto water and the resulting precipitate filtered off. The solid was dried to obtain the desired product (2.15 g, quant. yield) as a light yellow solid, MS: m/e = 324.1 (M-H⁺).

### Step 2: Methyl 2-fluoro-3-iodo-6-methyl-5-nitrobenzoate

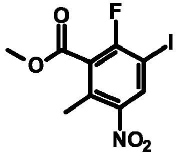

2-Fluoro-3-iodo-6-methyl-5-nitrobenzoic acid *(Example 7*, *step 1)* (2.15 g, 6.61 mmol) was dissolved in 10 ml of DMF. Potassium carbonate (2.0 g, 14.6 mmol, 2.2 equiv.) and iodomethane (1.03 g, 7.28 mmol, 1.1 equiv.) were added at room temperature. The mixture was stirred at room temperature for 2 hours. The reaction mixture was extracted with saturated sodiumbicarbonate solution and two times with TBME. The organic layers were extracted with water and 10% lithium chloride solution. The organic layers were combined, dried over sodium sulfate and concentrated to dryness to obtain the desired methyl 2-fluoro-3-iodo-6-methyl-5-nitrobenzoate (2.32 g, 98 % yield) as a lighty yellow solid, MS: m/e = 338.0 (M+H⁺).

### Step 3: Methyl 3-amino-6-fluoro-5-iodo-2-methylbenzoate

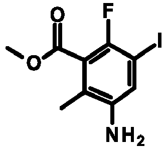

Methyl 2-fluoro-3-iodo-6-methyl-5-nitrobenzoate *(Example 7*, *step 2)* (2.32 g, 6.85 mmol) was dissolved in 50 ml of methanol and 25 ml of water. Ammonium chloride (3.67 g, 68.5 mmol, 10 equiv.) and iron (3.06 g, 54.8 mmol, 8 equiv.) were added at room temperature. The mixture was stirred at 70°C for 2 hours. The reaction mixture was filtered and evaporated to dryness. The residue was extracted with saturated sodiumbicarbonate solution and two times with ethyl acetate. The organic layers were extracted with water and brine. The organic layers were combined, dried over sodium sulfate and concentrated to dryness to obtain the desired methyl 3-amino-6-fluoro-5-iodo-2-methylbenzoate (2.22 g, quantitative yield) as a yellow oil, MS: m/e = 310.0 (M+H⁺).

### Step 4: Methyl 2,5-difluoro-3-iodo-6-methyl-benzoate

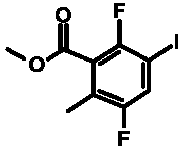

Methyl 3-amino-6-fluoro-5-iodo-2-methylbenzoate *(Example 7*, *step 3)* (2.2 g, 7.21 mmol) was dissolved in 20 ml of toluene. Nitrosonium tetrafluoroborate (1.26 g, 10.8 mmol, 1.5 equiv.) was added in portion and under ice cooling at room temperature. The mixture was stirred at room temperature for 10 minutes and at 110°C for 10 minutes. The reaction mixture was poured onto water and extracted twice with ethyl acetate. The organic layers were extracted with brine, dried over sodium sulfate and concentrated to dryness. The crude product was purified by flash chromatography on a silica gel column eluting with a heptane:ethyl acetate 100:0 to 80:20 gradient to obtain the desired product (1.36 g, 43 % yield) as a yellow oil.

### Step 5: Methyl 2-(bromomethyl)-3,6-difluoro-5-iodo-benzoate

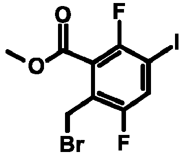

The title compound was obtained as a colorless solid, MS: m/e = 409.0 (M+H⁺), using chemistry similar to that described in Example 1, step 3 starting from methyl 2,5-difluoro-3-iodo-6-methyl-benzoate *(Example 7*, *step 4).*

### Step 6: Ethyl (2RS)-2-(4,7-difluoro-6-iodo-1-oxo-isoindolin-2-yl)-2-(6,7-dihydro-5H-pyrrolo[1,2-c]imidazol-1-yl)acetate

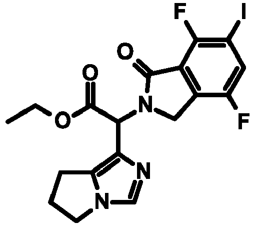

The title compound was obtained as a brown oil, MS: m/e = 488.1 (M+H⁺), using chemistry similar to that described in Example 2, step 1 starting from ethyl (2*RS*)-2-amino-2-(6,7-dihydro-SH-pyrrolo[1,2-c]imidazol-1-yl)acetate hydrochloride *(Example 1*, *step 4)* and methyl 2-(bromomethyl)-3,6-difluoro-5-iodo-benzoate *(Example 7*, *step 5).*

### Step 7: Ethyl (2RS)-2-[4,7-difluoro-6-[4-[3-[[4-(hydroxymethyl)-1-piperidyl]methyl]-1-bicyclo[1.1.1]pentanyl]phenyl]-1-oxo-isoindolin-2-yl]-2-(6,7-dihydro-5H-pyrrolo[1,2-c]imidazol-1-yl)acetate

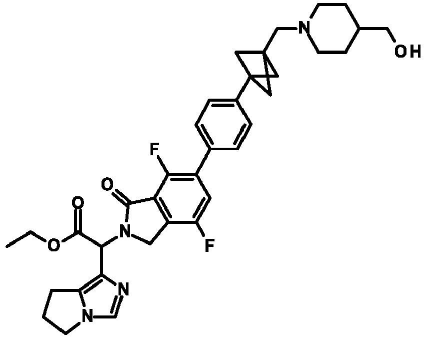

The title compound was obtained as a white foam, MS: m/e = 631.5 (M+H⁺), using chemistry similar to that described in Example 1, step 11 starting from ethyl (2RS)-2-(4,7-difluoro-6-iodo-1-oxo-isoindolin-2-yl)-2-(6,7-dihydro-5H-pyrrolo[1,2-c]imidazol-1-yl)acetate *(Example 7, step 6)* and and [1-[[3-[4-(4,4,5,5-tetramethyl-1,3,2-dioxaborolan-2-yl)phenyl]-1-bicyclo[1.1.1]pentanyl]methyl]-4-piperidyl]methanol *(Example 1*, *step 10).*

### Step 8: (2RS)-2-[4,7-Difluoro-6-[4-[3-[[4-(hydroxymethyl)-1-piperidyl]methyl]-1-bicyclo[1.1.1]pentanyl]phenyl]-1-oxo-isoindolin-2-yl]-2-(6,7-dihydro-5H-pyrrolo[1,2-c]imidazol-1-yl)-N-thiazol-2-yl-acetamide

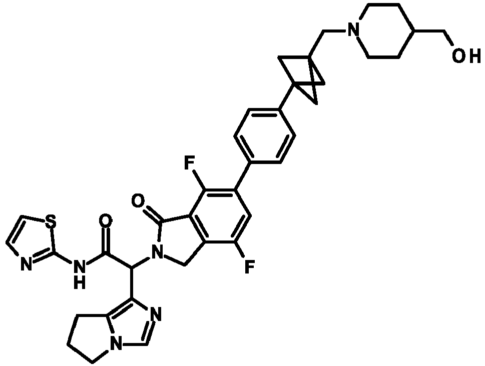

The title compound was obtained as a white foam, MS: m/e = 685.5 (M+H⁺), using chemistry similar to that described in Example 1, step 12 starting from ethyl (2RS)-2-[4,7-difluoro-6-[4-[3-[[4-(hydroxymethyl)-1-piperidyl]methyl]-1-bicyclo[1.1.1]pentanyl]phenyl]-1-oxo-isoindolin-2-yl]-2-(6,7-dihydro-SH-pyrrolo[1,2-c]imidazol-1-yl)acetate *(Example 7*, *step 7)* and thiazol-2-amine.

## Claims

1. A compound of formula (I) 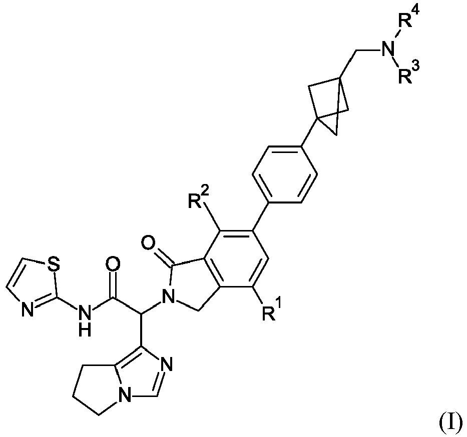 wherein
R¹ is selected from
i) fluoro, and
ii) chloro;
R² is selected from
i) H, and
ii) fluoro;
R³ is C₁₋₆-alkyl;
R⁴ is C₁₋₆-alkyl;
or R³ and R⁴ together with the nitrogen atom to which they are attached form an heterocycloalkyl optionally substituted with R⁵; and
R⁵ is selected from
i) hydroxy, and
ii) hydrox-C₁₋₆-alkyl;
or a pharmaceutically acceptable salt thereof.

2. A compound of formula (I) according to claim 1, wherein
R³ and R⁴ are methyl;
or R³ and R⁴ together with the nitrogen atom to which they are attached form an heterocycloalkyl optionally substituted with R⁵, wherein the heterocycloalkyl is selected from
i) piperidinyl,
i) pyrrolidinyl, and
ii) azepanyl; and
R⁵ is selected from
i) hydroxy, and
ii) hydroxymethyl;
or a pharmaceutically acceptable salt thereof.

3. A compound of formula (I) according to claim 1 or 2, wherein
R¹ is fluoro;
R² is selected from
i) H, and
ii) fluoro;
R³ and R⁴ together with the nitrogen atom to which they are attached form an heterocycloalkyl optionally substituted with R⁵, wherein the heterocycloalkyl is selected from
i) piperidinyl,
ii) pyrrolidinyl, and
iii) azepanyl; and
R⁵ is selected from
i) hydroxy, and
ii) hydroxymethyl;
or a pharmaceutically acceptable salt thereof.

4. A compound of formula (I) according to anyone of claims 1 to 3, wherein R¹ is fluoro.

5. A compound of formula (I) according to anyone of claims 1 to 4, wherein R² is H.

6. A compound of formula (I) according to anyone of claims 1 to 5, wherein R³ and R⁴ together with the nitrogen atom to which they are attached form an heterocycloalkyl optionally substituted with R⁵, wherein the heterocycloalkyl is selected from
i) piperidinyl,
ii) pyrrolidinyl, and
iii) azepanyl.

7. A compound according to any one of claims 1 to 6, wherein the compound is selected from
(2RS)-2-(6,7-dihydro-5H-pyrrolo[1,2-c]imidazol-1-yl)-2-[4-chloro-6-[4-[3-[[4-(hydroxymethyl)-1-piperidyl]methyl]-1-bicyclo[1.1.1]pentanyl]phenyl]-1-oxo-isoindolin-2-yl]-N-thiazol-2-yl-acetamide;
(2RS)-2-(6,7-dihydro-5H-pyrrolo[1,2-c]imidazol-1-yl)-2-[4-fluoro-6-[4-[3-[[4-(hydroxymethyl)-1-piperidyl]methyl]-1-bicyclo[1.1.1]pentanyl]phenyl]-1-oxo-isoindolin-2-yl]-N-thiazol-2-yl-acetamide;
(2RS)-2-(6,7-dihydro-5H-pyrrolo[1,2-c]imidazol-1-yl)-2-[6-[4-[3-[(dimethylamino)methyl]-1-bicyclo[1. 1. 1]pentanyl]phenyl]-4-fluoro-1-oxo-isoindolin-2-yl]-N-thiazol-2-yl-acetamide;
(2RS)-2-(6,7-dihydro-5H-pyrrolo[1,2-c]imidazol-1-yl)-2-[4-fluoro-6-[(4-hydroxy-1-piperidyl)methyl]-1-bicyclo[1.1.1]pentanyl]phenyl]-1-oxo-isoindolin-2-yl]-N-thiazol-2-yl-acetamide;
(2RS)-2-(6,7-dihydro-5H-pyrrolo[1,2-c]imidazol-1-yl)-2-[4-fluoro-1-oxo-6-[4-[3-(pyrrolidin-1-ylmethyl)-1-bicyclo[1.1.1]pentanyl]phenyl]isoindolin-2-yl]-N-thiazol-2-yl-acetamide;
(2RS)-2-(6,7-dihydro-5H-pyrrolo[1,2-c]imidazol-1-yl)-2-[4-fluoro-6-[4-[3-[[(4RS)-4-hydroxyazepan-1-yl]methyl]-1-bicyclo[1.1.1]pentanyl]phenyl]-1-oxo-isoindolin-2-yl]-N-thiazol-2-yl-acetamide; and
(2RS)-2-[4,7-difluoro-6-[4-[3-[[4-(hydroxymethyl)-1-piperidyl]methyl]-1-bicyclo[1.1.1]pentanyl]phenyl]-1-oxo-isoindolin-2-yl]-2-(6,7-dihydro-5H-pyrrolo[1,2-c]imidazol-1-yl)-N-thiazol-2-yl-acetamide;
or a pharmaceutically acceptable salt thereof.

8. A compound according to anyone of claims 1 to 7 for use as therapeutically active substance.

9. A pharmaceutical composition comprising a compound according to anyone of claims 1 to 7 and a therapeutically inert carrier.

10. A compound according to anyone of claims 1 to 7 for use in the treatment or prophylaxis of cancer.

11. A compound for use according to claim 10 wherein the cancer is non-small cell lung cancer.

## Patentansprüche

1. Verbindung der Formel (I) 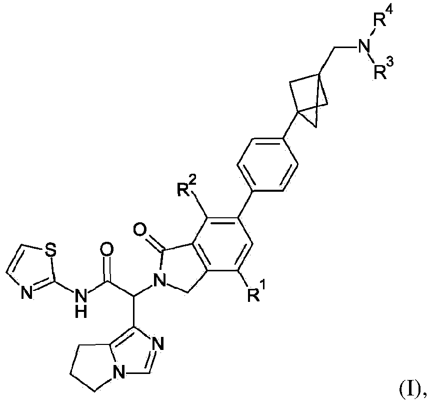 wobei
R¹ aus
i) Fluor und
ii) Chlor
ausgewählt ist;
R² aus
i) H und
ii) Fluor
ausgewählt ist;
R³ C₁₋₆-Alkyl ist;
R⁴ C₁₋₆-Alkyl ist;
oder R³ und R⁴ zusammen mit dem Stickstoffatom, an das sie gebunden sind, ein Heterocycloalkyl bilden, das gegebenenfalls mit R⁵ substituiert ist; und
R⁵ aus
i) Hydroxy und
ii) Hydrox-C₁₋₆-alkyl
ausgewählt ist;
oder ein pharmazeutisch unbedenkliches Salz davon.

2. Verbindung der Formel (I) nach Anspruch 1, wobei
R³ und R⁴ Methyl sind;
oder R³ und R⁴ zusammen mit dem Stickstoffatom, an das sie gebunden sind, ein Heterocycloalkyl bilden, das gegebenenfalls mit R⁵ substituiert ist, wobei das Heterocycloalkyl aus
i) Piperidinyl,
i) Pyrrolidinyl und
ii) Azepanyl
ausgewählt ist; und
R⁵ aus
i) Hydroxy und
ii) Hydroxymethyl
ausgewählt ist;
oder ein pharmazeutisch unbedenkliches Salz davon.

3. Verbindung der Formel (I) nach Anspruch 1 oder 2, wobei
R¹ Fluor ist;
R² aus
i) Hund
ii) Fluor
ausgewählt ist;
R³ und R⁴ zusammen mit dem Stickstoffatom, an das sie gebunden sind, ein Heterocycloalkyl bilden, das gegebenenfalls mit R⁵ substituiert ist, wobei das Heterocycloalkyl aus
i) Piperidinyl,
ii) Pyrrolidinyl und
iii) Azepanyl
ausgewählt ist; und
R⁵ aus
i) Hydroxy und
ii) Hydroxymethyl
ausgewählt ist;
oder ein pharmazeutisch unbedenkliches Salz davon.

4. Verbindung der Formel (I) nach einem der Ansprüche 1 bis 3, wobei R¹ Fluor ist.

5. Verbindung der Formel (I) nach einem der Ansprüche 1 bis 4, wobei R² H ist.

6. Verbindung der Formel (I) nach einem der Ansprüche 1 bis 5, wobei R³ und R⁴ zusammen mit dem Stickstoffatom, an das sie gebunden sind, ein Heterocycloalkyl bilden, das gegebenenfalls mit R⁵ substituiert ist, wobei das Heterocycloalkyl aus
i) Piperidinyl
ii) Pyrrolidinyl und
iii) Azepanyl
ausgewählt ist.

7. Verbindung nach einem der Ansprüche 1 bis 6, wobei die Verbindung ausgewählt ist aus
(2RS)-2-(6,7-Dihydro-5H-pyrrolo[1,2-c]imidazol-1-yl)-2-[4-chlor-6-[4-[3-[[4-(hydroxymethyl)-1-piperidyl]methyl]-1-bicyclo[1.1.1]pentanyl]phenyl]-1-oxoisoindolin-2-yl]-N-thiazol-2-ylacetamid;
(2RS)-2-(6,7-Dihydro-5H-pyrrolo[1,2-c]imidazol-1-yl)-2-[4-fluor-6-[4-[3-[[4-(hydroxymethyl)-1-piperidyl]methyl]-1-bicyclo[1.1.1]pentanyl]phenyl]-1-oxoisoindolin-2-yl]-N-thiazol-2-ylacetamid;
(2RS)-2-(6,7-Dihydro-5H-pyrrolo[1,2-c]imidazol-1-yl)-2-[6-[4-[3-[(dimethylamino)methyl]-1-bicyclo[1.1.1]pentanyl]phenyl]-4-fluor-1-oxoisoindolin-2-yl]-N-thiazol-2-ylacetamid;
(2RS)-2-(6,7-Dihydro-5H-pyrrolo[1,2-c]imidazol-1-yl)-2-[4-fluor-6-[4-[3-[(4-hydroxy-1-piperidyl)methyl]-1-bicyclo[1.1.1]pentanyl]phenyl]-1-oxoisoindolin-2-yl]-N-thiazol-2-ylacetamid;
(2RS)-2-(6,7-Dihydro-5H-pyrrolo[1,2-c]imidazol-1-yl)-2-[4-fluor-1-oxo-6-[4-[3-(pyrrolidin-1-ylmethyl)-1-bicyclo[1.1.1]pentanyl]phenyl]isoindolin-2-yl]-N-thiazol-2-ylacetamid;
(2RS)-2-(6,7-Dihydro-5H-pyrrolo[1,2-c]imidazol-1-yl)-2-[4-fluor-6-[4-[3-[[(4RS)-4-hydroxyazepan-1-yl]methyl]-1-bicyclo[1.1.1]pentanyl]phenyl]-1-oxoisoindolin-2-yl]-N-thiazol-2-ylacetamid; und
(2RS)-2-[4,7-Difluor-6-[4-[3-[[4-(hydroxymethyl)-1-piperidyl]methyl]-1-bicyclo[1.1.1]pentanyl]phenyl]-1-oxoisoindolin-2-yl]-2-(6,7-dihydro-5H-pyrrolo[1,2-c]imidazol-1-yl)-N-thiazol-2-ylacetamid;
oder ein pharmazeutisch unbedenkliches Salz davon.

8. Verbindung nach einem der Ansprüche 1 bis 7 zur Verwendung als therapeutisch wirksame Substanz.

9. Pharmazeutische Zusammensetzung, umfassend eine Verbindung nach einem der Ansprüche 1 bis 7 und einen therapeutisch inerten Träger.

10. Verbindung nach einem der Ansprüche 1 bis 7 zur Verwendung bei der Behandlung oder Prophylaxe von Krebs.

11. Verbindung zur Verwendung nach Anspruch 10, wobei der Krebs nicht-kleinzelliger Lungenkrebs ist.

## Revendications

1. Composé de formule (I) 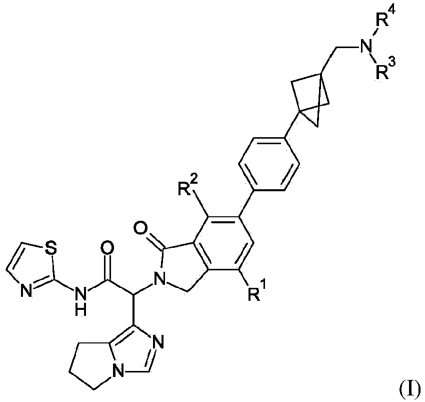 dans lequel
R¹ est choisi parmi
i) un fluor, et
ii) un chlore ;
R² est choisi parmi
i) H, et
ii) un fluor;
R³ représente un alkyle en C₁₋₆ ;
R⁴ représente un alkyle en C₁₋₆ ;
ou R³ et R⁴ conjointement avec l'atome d'azote auquel ils sont liés forment un hétérocycloalkyle éventuellement substitué par R⁵ ; et
R⁵ est choisi parmi
i) un hydroxy, et
ii) un hydroxyalkyle en C₁₋₆ ;
ou un sel pharmaceutiquement acceptable de celui-ci.

2. Composé de formule (I) selon la revendication 1, dans lequel
R³ et R⁴ représentent un méthyle ;
ou R³ et R⁴ conjointement avec l'atome d'azote auquel ils sont liés forment un hétérocycloalkyle éventuellement substitué par R⁵, dans lequel l'hétérocycloalkyle est choisi parmi
i) un pipéridinyle,
i) un pyrrolidinyle, et
ii) un azépanyle ; et
R⁵ est choisi parmi
i) un hydroxy, et
ii) un hydroxyméthyle ;
ou un sel pharmaceutiquement acceptable de celui-ci.

3. Composé de formule (I) selon la revendication 1 ou 2, dans lequel
R¹ représente un fluor ;
R² est choisi parmi
i) H, et
ii) un fluor ;
R³ et R⁴ conjointement avec l'atome d'azote auquel ils sont liés forment un hétérocycloalkyle éventuellement substitué par R⁵, dans lequel l'hétérocycloalkyle est choisi parmi
i) un pipéridinyle,
ii) un pyrrolidinyle, et
iii) un azépanyle ; et
R⁵ est choisi parmi
i) un hydroxy, et
ii) un hydroxyméthyle ;
ou un sel pharmaceutiquement acceptable de celui-ci.

4. Composé de formule (I) selon l'une quelconque des revendications 1 à 3, dans lequel R¹ représente un fluor.

5. Composé de formule (I) selon l'une quelconque des revendications 1 à 4, dans lequel R² représente H.

6. Composé de formule (I) selon l'une quelconque des revendications 1 à 5, dans lequel R³ et R⁴ conjointement avec l'atome d'azote auquel ils sont liés forment un hétérocycloalkyle éventuellement substitué par R⁵, dans lequel l'hétérocycloalkyle est choisi parmi
i) un pipéridinyle,
ii) un pyrrolidinyle, et
iii) un azépanyle.

7. Composé selon l'une quelconque des revendications 1 à 6, dans lequel le composé est choisi parmi
le (2RS)-2-(6,7-dihydro-SH-pyrrolo[1,2-c]imidazol-1-yl)-2-[4-chloro-6-[4-[3-[[4-(hydroxyméthyl)-1-pipéridyl]méthyl]-1-bicyclo[1.1.1]pentanyl]phényl]-1-oxo-isoindolin-2-yl]-N-thiazol-2-yl-acétamide ;
le (2RS)-2-(6,7-dihydro-5H-pyrrolo[1,2-c]imidazol-1-yl)-2-[4-fluoro-6-[4-[3-[[4-(hydroxyméthyl)-1-pipéridyl]méthyl]-1-bicyclo[1.1.1]pentanyl]phényl]-1-oxo-isoindolin-2-yl]-N-thiazol-2-yl-acétamide ;
le (2RS)-2-(6,7-dihydro-5H-pyrrolo[1,2-c]imidazol-1-yl)-2-[6-[4-[3-[(diméthylamino)méthyl]-1-bicyclo[1.1.1]pentanyl]phényl]-4-fluoro-1-oxo-isoindolin-2-yl]-N-thiazol-2-yl-acétamide ;
le (2RS)-2-(6,7-dihydro-5H-pyrrolo[1,2-c]imidazol-1-yl)-2-[4-fluoro-6-[4-[3-[(4-hydroxy-1-pipéridyl)méthyl]-1-bicyclo[1.1.1]pentanyl]phényl]-1-oxo-isoindolin-2-yl]-N-thiazol-2-yl-acétamide ;
le (2RS)-2-(6,7-dihydro-5H-pyrrolo[1,2-c]imidazol-1-yl)-2-[4-fluoro-1-oxo-6-[4-[3-(pyrrolidin-1-ylméthyl)-1 -bicyclo[1.1.1]pentanyl]phényl]isoindolin-2-yl]-N-thiazol-2-yl-acétamide ;
le (2RS)-2-(6,7-dihydro-5H-pyrrolo[1,2-c]imidazol-1-yl)-2-[4-fluoro-6-[4-[3-[[(4RS)-4-hydroxyazépan-1-yl]méthyl]-1-bicyclo[1.1.1]pentanyl]phényl]-1-oxo-isoindolin-2-yl]-N-thiazol-2-yl-acétamide ; et
le (2RS)-2-[4,7-difluoro-6-[4-[3-[[4-(hydroxyméthyl)-1-pipéridyl]méthyl]-1-bicyclo[1.1.1]pentanyl]phényl]-1-oxo-isoindolin-2-yl]-2-(6,7-dihydro-5H-pyrrolo[1,2-c]imidazol-1-yl)-N-thiazol-2-yl-acétamide ;
ou un sel pharmaceutiquement acceptable de celui-ci.

8. Composé selon l'une quelconque des revendications 1 à 7 pour une utilisation comme substance thérapeutiquement active.

9. Composition pharmaceutique comprenant un composé selon l'une quelconque des revendications 1 à 7 et un véhicule thérapeutiquement inerte.

10. Composé selon l'une quelconque des revendications 1 à 7 pour une utilisation dans le traitement ou la prophylaxie d'un cancer.

11. Composé pour une utilisation selon la revendication 10 dans lequel le cancer est un cancer du poumon non à petites cellules.
